(19) Europäisches Patentamt
European Patent Office
Office européen des brevets

(11) EP 4 697 432 A1

# (12) EUROPEAN PATENT APPLICATION

published in accordance with Art. 153(4) EPC

(43) Date of publication:
**18.02.2026 Bulletin 2026/08**

(21) Application number: **24787934.9**

(22) Date of filing: **29.03.2024**

(51) International Patent Classification (IPC):
***H01M 10/058*** *(2010.01)*

(52) Cooperative Patent Classification (CPC):
Y02E 60/10

(86) International application number:
**PCT/CN2024/084855**

(87) International publication number:
**WO 2024/212829 (17.10.2024 Gazette 2024/42)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **14.04.2023 CN 202310401041**

(71) Applicant: **Zhejiang Yongtai Technology Co., Ltd.**
**Taizhou, Zhejiang 317016 (CN)**

(72) Inventors:
- **GAO, Yue**
  **Shanghai 200433 (CN)**
- **CHEN, Shu**
  **Shanghai 200433 (CN)**

(74) Representative: **Winter, Brandl - Partnerschaft mbB**
**Alois-Steinecker-Straße 22**
**85354 Freising (DE)**

## (54) PRE-EMBEDDING AGENT, PRE-EMBEDDED LITHIUM POSITIVE ELECTRODE, PRE-EMBEDDED SODIUM POSITIVE ELECTRODE, SECONDARY BATTERY COMPRISING SAME, METHOD FOR PRE-EMBEDDING LITHIUM, AND METHOD FOR PRE-EMBEDDING SODIUM

(57) The present invention provides a pre-embedding agent using organic sulfinate as a framework for pre-embedding lithium or sodium, a pre-embedded lithium positive electrode, a pre-embedded sodium positive electrode, a secondary battery, and a method for pre-embedding lithium or sodium using the pre-embedding agent. The pre-embedding agent of the present invention has a high specific capacity, excellent stability, is free of residues, has excellent compatibility with a battery system, and can be well applied to lithium or sodium ion secondary batteries. A pre-embedding process of the method for pre-embedding lithium or sodium of the present invention is safe and convenient, the process is simple, the costs are low, and large-scale production can be achieved.

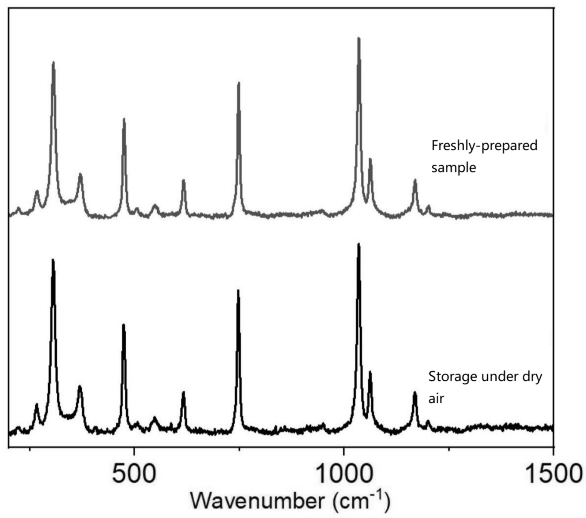


FIG.4

EP 4 697 432 A1

## Description

## TECHNICAL FIELD

The disclosure relates to a pre-embedding agent, a pre-embedded lithium positive electrode containing a pre-embedded lithium material, a secondary battery containing the pre-embedded lithium positive electrode, and a method for pre-embedding lithium. In addition, the present disclosure also relates to a pre-embedding sodium positive electrode containing a pre-embedded sodium material, a secondary battery containing the pre-embedded sodium positive electrode, and a method for pre-embedding sodium.

## BACKGROUND ART

In view of the increasing energy demand and severe environmental problems, it is urgent to develop efficient and reliable energy storage technology. Lithium/sodium battery technology is considered to be one of the most promising energy storage technologies. With its advantages such as high energy density and low cost, it has been widely used in electric vehicles and portable electric devices. However, with the further development of human society, various new applications have further increased requirements for energy storage technology, and current lithium/sodium battery technology faces the problem of insufficient energy density. Therefore, for lithium/sodium batteries, the use of anode materials with high capacity density needs to be investigated, for example, new lithium batteries such as silicon (4200 mAh g-1) and phosphorus (2596 mAh g-1). However, these negative electrode materials are accompanied by irreversible side reactions during the charge/discharge process, resulting in the loss of lithium ions and the attenuation of battery capacity. For example, silicon-based materials (SiOx) undergo irreversible electrochemical reactions to form LiySiOx, which cannot be reused, in addition, the formation of a solid electrolyte interphase (SEI) on the surface of the silicon negative electrode will also consume some lithium ions. During the subsequent charging process, the lithium ions that can migrate back to the positive electrode are reduced, resulting in loss of battery capacity and inability to fully utilize the positive electrode material. These side reactions result in the initial coulombic efficiency (ICE) and cycle efficiency (CE) of the battery being far below 100%. Taking SiO negative electrode as an example, its full cell ICE is only 50%-60%, which means that the battery loses more than 40% of its capacity in the first cycle, greatly limiting the further development of these materials. Therefore, it is necessary to solve this problem through the method of pre-embedding lithium, that is, before assembling the battery, a certain amount of lithium is stored in the battery in advance to compensate for the lithium loss in the initial cycle of the battery. As for sodium batteries, the above problems also exist. However, there are no pre-embedding sodium agent and method in the industry for stable, effective, and beneficial sodium compensation.

Currently, the more mature pre-embedding lithium technologies mainly include two approaches: directly introducing lithium sources through the negative electrode and adding them through the positive electrode. Regarding the technology of directly introducing lithium sources through the negative electrode, although its pre-embedding efficiency of lithium is high and no invalid components are introduced, it has high process requirements, high cost, poor safety, and is not conducive to large-scale production. Although the pre-embedding lithium technology of adding through the positive electrode has the advantages of high safety and simple process requirements, by-products will remain in the battery after lithium compensation, which will reduce the energy density of the battery and generate pores in positive electrode, thereby reducing the battery's cyclability. Therefore, there is an urgent need for a positive electrode lithium compensation technology that will not leave any by-products in the battery after lithium compensation and will increase the energy density of battery.

Patent Document 1 discloses a pre-lithiation method of lithium-sulfur battery electrodes. By pre-lithiating the sulfur positive electrode, the introduction of a lithium source into the negative electrode can be avoided. Since the sulfur positive electrode has low conductivity, heat release is relatively slow during contact with the lithium source, which helps to reduce hazards and improve safety performance. The use of lithium foil to lithiate the positive electrode can avoid the introduction of other substances, which helps to ensure energy density and is suitable for battery systems that lack all lithium sources. However, the above-mentioned Patent Document 1 requires the use of lithium metal materials, which are unstable in air and inconvenient to operate. In addition, the short-circuit lithium compensation method involves a violent reaction and is difficult to control reaction time, which will lead to instability of the positive electrode interface and affect the battery cycle performance.

Patent document 2 discloses a double-layer composite diaphragm battery and a lithium compensation method thereof, which can effectively avoid uncontrollable reaction speed during lithium compensation, achieve the purpose of uniform lithium compensation, and greatly improve the battery's initial cycle charge/discharge efficiency and energy density. However, in Patent Document 2, a three-electrode system containing lithium metal is used for lithium compensation of the battery. This method also requires the use of lithium metal materials and the filling of lithium metal electrodes between the double diaphragms. It has disadvantage of air instability and inconvenient operation. The introduction of additional components will reduce the energy density of the battery system.

Patent Document 3 discloses a method of using a sodium compensation additive $Na_2C_xO_yN_z$ mixed with a positive electrode material in a certain ratio (5% to 20%) to form mixed positive electrode material for sodium compensation of sodium ion batteries. However, the materials required for this method need to be decomposed at high temperatures, which is costly, and the remaining components after sodium compensation are buried in the battery, reducing the overall energy density of the battery.

Other existing technologies include using sodium compensation additives such as $Na_5FeO_4$ or $Na_2C_2O_4$ mixed with positive electrode materials in a certain proportion to form mixed positive electrode materials for sodium compensation of sodium ion batteries. Similarly, the materials required for these methods need to undergo high voltage decomposition, which will reduce the stability of the battery interface and affect the long-term cycle use of the battery. After sodium compensation, the remaining components are buried in the battery, which reduces the overall energy density of the battery.

Existing technology

Patent Document 1: CN116470165A
Patent Document 2: CN107845829A
Patent Document 3: CN110165218A.

## SUMMARY

Technical problem to be solved by the disclosure

The object of the present disclosure is to provide a pre-embedding agent, a pre-embedded lithium/sodium positive electrode, a lithium/sodium ion battery containing the same, and a pre-embedding lithium/sodium method, which remains no by-products in the battery after pre-embedding lithium/sodium, has good stability, a safe and convenient pre-embedding lithium/sodium process, and a high energy density and capacity retention rate.

Technical means to solve the problem

The present disclosure provides an organic pre-embedding agent which has little or even no residue after pre-embedding treatment. The pre-embedded lithium/sodium positive electrode of the present disclosure is obtained by treating with the organic pre-embedding agent of the present disclosure. The pre-embedding lithium/sodium method of the present disclosure is a method of lithium/sodium pre-embedding of the positive electrode using the organic pre-embedding agent of the present disclosure.

Beneficial technical effects

The side groups of the organic pre-embedding agent of the present disclosure are adjustable, so that the structure of material can be designed by selecting different side groups, and its synthesis process is simple and the cost is low. In addition, the pre-embedded lithium/sodium via the organic pre-embedding agent has a high specific capacity and a moderate electric potential of lithium/sodium de-intercalation, and has good compatibility with the battery system. The pre-embedding agent of the present disclosure has good stability, a safe and convenient lithium/sodium pre-embedding process, few residual components in the battery system, no influence on the battery cycle performance, a simple production process, low cost, and can be produced on a large scale.

## BRIEF DESCRIPTION OF THE DRAWINGS

FIG. 1 is a CV test curve and a charge/discharge test curve of the sodium trifluoromethanesulfinate coin cell in Example 1 of the present disclosure.
FIG. 2 is a CV test curve and a charge/discharge test curve of the lithium trifluoromethanesulfinate coin cell in Example 1 of the present disclosure.
FIG. 3 is an in-situ Raman characterization of the lithium pre-embedding method of lithium trifluoromethanesulfinate electrolyte in Example 1 of the present disclosure, and a Raman comparison of lithium trifluoromethanesulfinate powder, pure electrolyte, and lithium trifluoromethanesulfinate dissolved in pure electrolyte after charging.
FIG. 4 is a Raman comparison of a fresh sample of lithium trifluoromethanesulfinate and a sample stored in dry air in

Example 1 of the present disclosure.

FIG. 5 is a charge/discharge curve test of pre-embedding agents with different side group substitutions in Examples 2, 3, 4, and 5 of the present disclosure.

FIG. 6 is a charge/discharge curve test of sodium organic sulfinates with different substituents according to the present disclosure.

FIG. 7 is a diagram showing the charge/discharge efficiency in Example 8 of the present disclosure.

FIG. 8 is a diagram showing the charge/discharge efficiency in Example 9 of the present disclosure.

FIG. 9 is a comparison diagram of the lithium compensation effect in Example 10 of the present disclosure.

FIG. 10 is a comparison diagram of the electrolyte sodium compensation effect in Example 11 of the present disclosure.

FIG. 11 is a comparison diagram of the effect of sodium compensation by coating the positive electrode surface in Example 12 of the present disclosure.

FIG. 12 is a comparison diagram of the effects of sodium compensation by doping the positive electrode in Example 13 of the present disclosure.

FIG. 13 is a comparison diagram of the effects before and after sodium pre-embedding in Example 14 of the present disclosure.

FIG. 14 is a diagram showing the efficiency of multiple electrolyte lithium compensation in Example 15 of the present disclosure.

## DETAILED DESCRIPTION

The present disclosure is described in detail below, but the present disclosure is not limited thereto and any changes can be made without departing from the scope of the present disclosure.

[pre-embedding agent]

The pre-embedding agent of the present disclosure has an organic sulfinate structure as shown below:

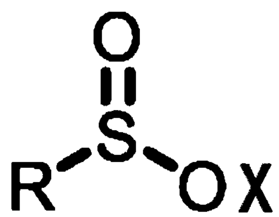

formula (1)

wherein, in the above formula (1), R is selected from an alkyl group or a halogenated alkyl group having 1 to 10 carbon atoms, an aryl group or a halogenated aryl group having a benzene ring, a thiophene ring, a furan ring, or a pyridine ring, a chain ether group or a cyclic ether group having 1 to 10 carbon atoms, a chain ester group or a cyclic ester group having 1 to 10 carbon atoms. X represents Na or Li.

Preferably, R is an alkyl group selected from -Me, -Et, a halogenated alkyl group selected from $-CF_3$, $-C_2F_5$, $-C_4F_9$, an aryl group selected from -Ph, $-CH_3C_6H_4$, $-C_5NH_4$, $-C_4H_3S$, $-C_4H_3O$ ; and a halogenated aryl group selected from $-FC_6H_4$, $-ClC_6H_4$.

In the present disclosure, when X is Na, sodium compensation can be performed for sodium-ion secondary batteries, and when it is Li, lithium compensation can be performed for lithium-ion secondary batteries. The capacities of the secondary batteries after pre-embedding treatment can reach 311 mAh/g and 262 mAh/g, respectively, and have a moderate lithium/sodium desorption potential of 3.2 V-4.1 V.

The pre-embedding agent of the present disclosure has the following reactions in the process of pre-embedding lithium/sodium:

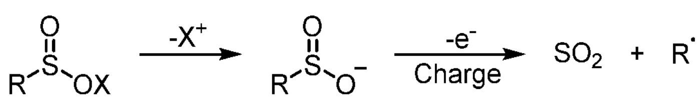

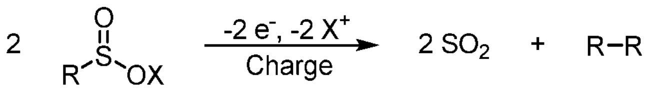


As shown above, the positive and negative ions in organic sulfinate salts are balanced. When the negative ions lose electrons and are oxidized and decomposed, the positive ions are released, achieving lithium compensation and sodium compensation. This process is only related to the oxidation potential of the negative ion, which is lower than 4.3 V relative to lithium metal and lower than 4 V relative to sodium metal, both of which are within the normal operating voltage range of lithium batteries or sodium batteries. Therefore, X can be either lithium or sodium. Other organic structures, such as organic sulfonates, organic carboxylates, organic pentafluorophosphates, organic trifluoroborates, organic phenolates, etc. cannot achieve the effects of the present disclosure, since the negative ion oxidation potential is not within the normal operating voltage range of lithium batteries or sodium batteries as for lithium metal or sodium metal.

Furthermore, the side groups of the organic pre-embedding agent can be adjusted by selecting R, thereby adjusting different parameters such as voltage, solubility and product to meet the needs of different battery systems. For example, as shown in the above reaction formula, when R is methyl or ethyl, the capacity per gram of the pre-embedding agent is the highest, the product is all gas, the decomposition voltage is low, but the solubility is poor, so it is suitable for use by blade coating on the surface of the positive electrode or for doping inside the positive electrode. When R is a fluoroalkyl group, the pre-embedding agent has a high solubility in the electrolyte and is therefore suitable for use in the electrolyte. In particular, trifluoromethyl is more preferred from the perspectives of capacity per gram of pre-embedding agent, voltage and solubility

Since the organic sulfinate structure only occupies the mass and volume of active lithium ions or sodium ions in the battery, the pre-embedding agent of the present disclosure has high specific energy and volume energy density. When R is -Me, -Et, - $CF_3$, or -$C_2F_5$, besides generating active lithium ions or sodium ions, other products are gases that can be completely discharged from the battery system. When R is other groups, besides generating active lithium ions or sodium ions, other products are dissolved in the electrolyte to form co-solvents, which will not increase the overall volume of the battery. Therefore, it has a high volume energy density.

In the present disclosure, when pre-embedding lithium/sodium using the pre-embedding agent with the above-mentioned chemical structure of organic sulfinate, the by-products can be fully discharged as gases, so the residue in the electrode is small and the overall energy density of the battery is not reduced. Therefore, when using the electrolyte to pre-embed lithium/sodium, it has the characteristics of no residue and large compensation, which can achieve the effects of low cost, simple preparation and easy storage.

The pre-embedding agent with the chemical structure of organic sulfinate in the present disclosure can be directly obtained from commercial products. The lithium salt can also be obtained by a production method including the following steps.

Step (1): weighing the commercially available sodium organic sulfinate, placing in a solvent, and recrystallizing to obtain a pre-embedded sodium material;

Step (2): weighing the pre-embedded sodium material in the step (1), placing the pre-embedded sodium material in an organic solvent, dropwise adding an appropriate amount of acid, stirring to react, extracting and drying, and evaporating the organic solvent;

Step (3): placing the product obtained in the step (2) in deionized water, adding an appropriate amount of lithium salt, stirring to react, and evaporating the solvent;

Step (4): recrystallizing the solid obtained in the step (3) using ethyl acetate and dichloromethane, drying the obtained solid powder to obtain an lithium organic sulfinate salt as the pre-embedded lithium material.

Preferably, the solvent used in the above step (1) is selected from at least one of water, ethyl acetate and ethanol.

The organic solvent used in the above step (2) is selected from at least one of acetonitrile, tetrahydrofuran, diethyl ether or ethanol. These solvents are preferred due to their good water solubility.

The acid used in the above step (2) is selected from non-oxidative acids such as hydrochloric acid, sulfuric acid, or acetic acid.

The lithium salt used in the above step (3) is selected from water-soluble lithium salts such as lithium hydroxide, lithium carbonate, or lithium acetate.

Preferably, in the above step (2), after adding the acid, the stirring reaction time is 1-10 hours. In step (3), after adding the lithium salt, the stirring reaction time is 5-10 hours. The drying process in the above steps can preferably be vacuum drying, with the temperature is 50-100°C and the time is 10-24 hours.

[lithium pre-embedding method/sodium pre-embedding method]

As the lithium/sodium pre-embedding method of the present disclosure, specifically, the pre-embedding agent of

the present disclosure can be directly added to the electrolyte to carry out the electrochemical reaction of thorough charging oxidation decomposition, or it can be implemented by electrode doping method or coating on the electrode surface.

[lithium pre-embedding method/sodium pre-embedding method]

As the lithium/sodium pre-embedding method of the present disclosure, specifically, the pre-embedding agent of the present disclosure can be directly added to the electrolyte to carry out the electrochemical reaction of charging oxidation decomposition, or it can be implemented by electrode doping method or coating on the electrode surface.

The electrode doping method is as follows. The pre-embedding agent of the present disclosure is mixed with a positive electrode material, a conductive agent, a binder, and a solvent as needed, and then the mixture is uniformly ground using a grinder such as an agate bead mill. A coating machine is used to coat the electrode current collector to form a thin film, which is then vacuum-dried in a vacuum oven to obtain an electrode sheet containing the pre-embedding agent with a chemical structure of organic sulfinatein the present disclosure. The electrode sheet is assembled into a pre-packaged battery, which is electrified to carry out the above electrochemical reaction to perform a lithium/sodium pre-embedding process. Since the pre-embedding agent of the present disclosure can only generate gaseous by-products during electrochemical reaction, the by-products can be fully discharged as gas, thereby avoiding the introduction of additional components.

In addition to the above method, another advantage of the pre-embedding agent of the present disclosure is that it can be dissolved in conventional commercial battery electrolytes, and then the electrolyte is added to the battery to achieve pre-embedding lithium/sodium in lithium/sodium batteries. In this method, specifically, the above-mentioned pre-embedding agent is dissolved in the battery electrolyte at a mass fraction of 0.1%-30%, preferably 0.1%-6%, and then the electrolyte is added to the battery to achieve pre-embedding lithium/sodium in battery. In particular, the preferred battery electrolyte is a carbonate electrolyte or an ether electrolyte.

When lithium compensation or sodium compensation is performed through the electrolyte, the pre-embedding agent exists in the electrolyte system in a free ionic state. During charging, there will be a potential difference between the positive electrode and the negative electrode of the battery. Under the action of the electric potential difference, negative ions will be concentrated and adsorbed on the surface of the positive electrode for electrochemical oxidation, and X positive ions will be embedded in the negative electrode to achieve lithium compensation or sodium compensation.

The pre-embedding agent of the present disclosure and the lithium/sodium pre-embedding method using the pre-embedding agent are not only applicable to the above-mentioned electrode doping method and coating method, but also have the characteristics of being able to directly dissolve the pre-embedding agent in the battery electrolyte. Therefore, it has good compatibility with batteries and can be effectively applied to lithium batteries and sodium batteries. In addition, when directly dissolved in the electrolyte, it does not need to be used through positive electrode doping, and can be directly added through the electrolyte preparation, which can more effectively avoid the introduction of additional components and will not lead to a decrease in the battery energy density. In addition, for used batteries, especially waste lithium batteries, the electrolyte can be added multiple times to achieve multiple lithium/sodium pre-embedding in batteries, thereby realizing the *in-situ* regeneration of waste lithium batteries without unpacking. The lithium/sodium pre-embedding process by adding electrolyte will not change the structure of battery components, such as causing the positive electrode to be porous, and a more stable electrode which is pre-embedded with lithium/sodium can be obtained. In addition, the method of adding electrolyte to the battery is highly integrated with the battery production process, does not require additional costs, and has a low cost of use.

[Lithium-ion secondary battery electrode]

As the positive electrode/negative electrode of the lithium-ion secondary battery, for example, a positive electrode plate/negative electrode plate with a structure in which an active material layer containing a positive electrode active material/negative electrode active material and a binder is formed on a current collector can be used.

The positive electrode current collector and the negative electrode current collector of the present disclosure may be made of any material that can be used as a current collector for lithium ion batteries. For example, copper can be used as the negative electrode current collector, and aluminum can be used as the positive electrode current collector.

The positive electrode active material of the present disclosure can be any material that can be used as a positive electrode active material for lithium ion batteries, for example, organic sulfur positive electrodes such as sulfurized polyacrylonitrile (SPAN), etc., lithium iron phosphate, lithium cobalt oxide, lithium manganese oxide, titanium disulfide, vanadium pentoxide, Prussian blue ($Fe_4[Fe(CN)_6]_3$), and ternary materials such as NCM811.

The positive electrode may contain a binder. As the binder, any substance commonly used as a positive electrode binder for lithium ion batteries in the art, such as polyvinylidene difluoride (PVDF), polyacrylic acid-based binders, and polyurethane-based binders, can be used.

The negative electrode active material of the present disclosure can be any material that can be used as a negative electrode active material for a lithium ion battery, for example, lithium metal, graphite, silicon carbon, Si or a composite of these, such as a composite of Si and graphite, a composite of Si and lithium metal, a composite of graphite and lithium metal, etc., silicon oxide compounds such as silicon monoxide (SiO), etc.

The active material layer may further contain a conductive agent. Examples of the conductive agent include any substance commonly used as a conductive agent for lithium-ion batteries in the art, such as conductive carbon Super P, carbon nanotubes (CNTs), natural graphite, artificial graphite, cokes, carbon black, pyrolytic carbons, carbon fibers, and calcined organic polymer compounds.

The pre-embedded lithium positive electrode of the present disclosure can be subjected to a lithium pre-embedding treatment by an electrode doping method, a coating method, or a method where the pre-embedding agent is dissolved in an electrolyte and an electrochemical reaction is carried out to pre-embed lithium. The pre-embedded lithium/sodium electrode in the existing technology requires the use of lithium metal materials, which are unstable in air and inconvenient to operate, involve a violent reaction and are difficult to control reaction time, which will lead to instability of the positive electrode interface and affect the battery cycle performance. The pre-embedded lithium material with a chemical structure of organic sulfinate of the present disclosure is stable to water and oxygen, easy to operate, and has a mild reaction process, thereby effectively avoiding the above problems.

Calculated based on a 1Ah battery, the initial efficiency of traditional LFP-graphite battery system, ternary-graphite battery system and lithium cobalt oxide-graphite battery system is above 95%. To achieve 100% efficiency, the required amount of lithium compensation is between 0.1% and 5%. In special battery systems which do not contain lithium ions, such as lithium-free positive electrode-graphite battery system and lithium-free positive electrode-copper battery system, the initial efficiency is around 50% after lithium compensation to 100%. To achieve 100% efficiency, the required amount of lithium compensation is between 100% and 200%.

As described above, since the product after the pre-embedding treatment of the present disclosure is gaseous or soluble in the electrolyte, the amount of residue in the electrode is small and can be determined by the pre-embedding agent content in the electrode before and after the pre-embedding treatment. When the pre-embedded range of lithium for 1Ah battery capacity is 0.1%-200%, the pre-embedding agent content in the pre-embedded lithium positive electrode before lithium pre-embedding treatment is calculated by the following formula. The calculation for sodium battery is the same as that for lithium battery.

Specifically, based on a 1Ah battery capacity, the amount of pre-embedded lithium c relative to the 1Ah battery capacity is 1*X% (Ah), and the capacity of the lithium compensation agent is $Q = \frac{F \cdot N}{3.6 \cdot M}$ ( F is the Faraday constant ; M is the molecular weight of the lithium compensation agent; N is the number of lithium ions in the lithium compensation agent, which is 1 in the formula (1) of the present disclosure), the amount of substance of the lithium compensation agent is

$$n = \frac{c}{Q \cdot M} = \frac{c \cdot 3.6 \cdot M}{F \cdot N \cdot M} = \frac{c \cdot 3.6}{F}$$

(mol). Therefore, when F is taken as 96500, when calculated according to the above-mentioned conventional industrial pre-embedded lithium range of 0.1%-200%, that is, the pre-embedded lithium amount c relative to the 1Ah battery capacity is 1mAh-2000mAh, the minimum amount of the pre-embedding agent content in the pre-embedded lithium positive electrode before lithium pre-embedding treatment is 1*3.6*1000/96500=0.0374 mmol, and the maximum amount is 2000*3.6*1000/96500=74.8 mmol. The amount of pre-embedding agent remaining in the treated electrode can be determined by measuring the amount of residual anions in the electrolyte and subtracting the amount from the amount before the pre-embedding treatment, but the method is not limited thereto. Since the pre-embedding agent of the present disclosure can be completely free of residue, the content of the pre-embedding agent in the pre-embedded lithium positive electrode after the lithium pre-embedding treatment is theoretically substantially zero, and in actual industry, it only needs to be less than 0.005 mmol.

The pre-embedding agent with the above-mentioned chemical structure of organic sulfinate of the present disclosure can achieve lithium compensation in used batteries by adding electrolyte multiple times, thereby extending the life of lithium-ion batteries. As preferred combinations, examples may include a combination of graphite negative electrode and lithium iron phosphate positive electrode, a combination of graphite negative electrode and ternary positive electrode, ta combination of graphite negative electrode and lithium cobalt oxide positive electrode, a combination of graphite negative electrode and lithium manganese oxide positive electrode, a combination of silicon-carbon negative electrode and lithium iron phosphate positive electrode, a combination of silicon-carbon negative electrode and ternary positive electrode, a combination of silicon-carbon negative electrode and lithium cobalt oxide positive electrode, a combination of silicon-carbon negative electrode and lithium manganese oxide positive electrode, a combination of silicon negative electrode and lithium iron phosphate positive electrode, a combination of silicon negative electrode and ternary positive electrode, a combination of silicon negative electrode and lithium cobalt oxide positive electrode and a combination of

silicon negative electrode and lithium manganese oxide positive electrode. The capacity of the battery system decreases after multiple cycles. The method for pre-embedding lithium in the electrolyte of the present disclosure can achieve a 15-30 times increase in the life of the lithium-ion battery. By using the pre-embedding agent with the above-mentioned chemical structure of organic sulfinate of the present disclosure for lithium compensation in the battery system via the electrolyte, waste lithium batteries can be regenerated *in situ* without unpacking. Compared with the existing technology, the operation convenience, environmental protection and cost are greatly improved.

In addition, the pre-embedding agent with the above-mentioned chemical structure of organic sulfinate of the present disclosure has sufficiently high specific energy and volume energy density, and therefore is applicable to low initial-effect electrode materials such as silicon monoxide (SiO), silicon carbon, silicon, and black phosphorus. Although these low initial-effect electrode materials have high specific energy, stable cycle life and low cost, they cannot be applied in practice, since the initial coulombic efficiency is lower than 92% required by lithium batteries. However, by applying the pre-embedding agentwith the above-mentioned chemical structure of organic sulfinate of the present disclosure, a high energy density battery was successfully realized. In addition, the SiO material itself is stable to water and oxygen. Compared with the existing commercial graphite negative electrode, its stability and energy density are greatly improved, and the cost is greatly reduced. As positive electrode materials that are paired with low initial-effect electrode materials such as silicon monoxide (SiO), examples include preferably NCM811. Other combinations are listed as follows: a combination of a silicon-carbon negative electrode and a ternary positive electrode, a combination of a silicon-carbon negative electrode and a lithium iron phosphate positive electrode, a combination of a silicon-carbon negative electrode and a lithium cobalt oxide positive electrode, a combination of a silicon-carbon negative electrode and a lithium manganese oxide positive electrode, a combination of a silicon negative electrode and a ternary positive electrode, a combination of a silicon negative electrode and a lithium iron phosphate positive electrode, a combination of a silicon negative electrode and a lithium cobalt oxide positive electrode, a combination of a silicon negative electrode and a lithium manganese oxide positive electrode, a combination of a black phosphorus negative electrode and a ternary positive electrode, a combination of a black phosphorus negative electrode and a lithium iron phosphate positive electrode, a combination of a black phosphorus negative electrode and a lithium cobalt oxide positive electrode, a combination of a black phosphorus negative electrode and a lithium manganese positive electrode, a combination of a silicon monoxide negative electrode and a ternary positive electrode, a combination of a silicon monoxide negative electrode and a lithium iron phosphate positive electrode, a combination of a silicon monoxide negative electrode and a lithium cobalt oxide positive electrode, and a combination of a silicon monoxide negative electrode and a lithium manganese oxide positive electrode.

Furthermore, the pre-embedding agent having the above-mentioned chemical structure of organic sulfinate of the present disclosure is particularly suitable for battery systems in which both the positive and negative electrodes do not contain lithium due to its sufficiently high specific energy and volume energy density. Examples of such electrode combinations include a combination of graphite as a lithium-free negative electrode material and sulfurized polyacrylonitrile (SPAN) as a lithium-free positive electrode material, a combination of graphite as a lithium-free negative electrode material and a titanium disulfide positive electrode, a combination of graphite as a lithium-free negative electrode material and a vanadium pentoxide positive electrode, a combination of a silicon-carbon negative electrode and a sulfurized polyacrylonitrile positive electrode, a combination of a silicon-carbon negative electrode and a titanium disulfide positive electrode, a combination of a silicon-carbon negative electrode and a vanadium pentoxide positive electrode, a combination of a silicon negative electrode and a sulfurized polyacrylonitrile positive electrode, a combination of a silicon negative electrode and a titanium disulfide positive electrode, and a combination of a silicon negative electrode and a vanadium pentoxide positive electrode. By applying the pre-embedding agent of the present disclosure, the energy density reaches 360 Wh $kg^{-1}$, which is higher than the current lithium-ion batteries (such as lithium iron phosphate, lithium cobalt oxide and ternary material systems are 200-300 Wh $kg^{-1}$), and its capacity retention rate is still above 80% after 350 cycles of charge/discharge.

The above-mentioned lithium-free positive electrode material SPAN and graphite itself are stable to water, oxygen, etc. Compared with existing commercial lithium-containing positive electrode materials (such as lithium iron phosphate, lithium cobalt oxide and ternary materials), its stability and processing convenience are greatly improved. The lithium-free positive electrode material SPAN is an organic polymer material. Its preparation process does not require the use of transition metals. Compared with existing commercial lithium-containing positive electrode materials (such as lithium iron phosphate, lithium cobalt oxide and ternary materials), it has high renewability, low production and recycling costs, and is more environmentally friendly. The synthesis process of pre-embedded lithium materials does not require high temperature and high pressure conditions, and has high stability and low production and storage costs.

[sodium-ion secondary battery electrode]

The sodium-ion secondary battery electrode of the present disclosure can be obtained by the same method as the lithium-ion secondary battery electrode. The abovementioned materials for lithium-ion secondary battery electrode or sodium-ion secondary battery electrode can be used as a electrode material. For example, electrode combinations are

classified according to sodium positive electrode materials, and the sodium battery systems are listed as follows: polyanion sodium battery systems$(XO_4)^{n-}$ or $(X_mO_{3m+1})^{n-}$ (X=B, S, P, Si, As, Mo, W), layered oxide sodium battery systems $Na_xMO_2$ (x=0~1, M=Fe, Mn, Ni, Co, Cr) and their combinations, Prussian blue sodium battery systems {$Na_x$M1 $[M2(CN)_6]_{1-y}$ ▪ $\square_y$ ▪ $nH_2O$ (0≤x≤2, 0≤y<1), where M1 and M2 are transition metal ions with different coordination (where M1 is coordinated with N and M2 is coordinated with C), such as Mn, Fe, Co, Ni, Cu, Zn, Cr, etc.; □ is a $[M2(CN)_6]$vacancy}. Examples of the conductive carbon include Super P and acetylene black, etc. Examples of the binder include PVDF, PTFE, and carbon nanotubes, etc.

From the perspective of better air stability, lower synthesis cost and process difficulty, and greater suitability for large-scale preparation of sodium battery positive electrode materials, P2-type layered oxides ($Na_{2/3}M_xN_yO_2$, x+y=1, M, N=Fe, Mn, Ni, Co, Cr) are more preferred as positive electrode materials, and hard carbon is preferably used as the negative electrode material for combination with this positive electrode material. In addition, the sodium-poor P2-type layered oxide ($Na_{0.67}Mn_{0.67}Ni_{0.33}O_2$) material itself is stable to water, oxygen, etc., and its stability is greatly improved and its cost is greatly reduced compared with existing commercial sodium-rich positive electrode materials.

Examples of hard carbon include resin carbon, organic polymer pyrolytic carbon, and carbon black, which are commonly used materials in the art. Examples of hard carbon negative electrode precursor materials include biomass, resin-based materials, and asphalt, etc.

[Electrolyte]

As the lithium-ion electrolyte, an electrolyte prepared by dissolving the pre-embedding agent with the above-mentioned chemical structure of lithium organic sulfinate of the present disclosure in an organic solvent is used. In addition, an electrolyte consisting of other lithium salts dissolved in an organic solvent is also required. Examples of the lithium salt include $LiClO_4$, $LiPF_6$, $LiAsF_6$, $LiSbF_6$, $LiBF_4$, $LiSO_3F$, $LiCF_3SO_3$, $LiN(SO_2CF_3)_2$, LiTFSI, $LiN(SO_2C_2F_5)_2$, $LiN(SO_2CF_3)(COCF_3)$, $Li(C_4F_9SO_3)$, $LiC(SO_2CF_3)_3$, LiBOB, lower aliphatic carboxylic acid lithium salts, and $LiAlCl_4$. These may be used alone or as a mixture of two or more.

As the organic solvent, for example, carbonates such as propylene carbonate, ethylene carbonate (EC), dimethyl carbonate (DMC), diethyl carbonate (DEC), ethyl methyl carbonate (EMC), 4-trifluoromethyl-1,3-dioxolane-2-one, and 1,2-bis(methoxycarbonyloxy)ethane; ethers such as 1,3-dioxolane (DOL), 1,2-dimethoxyethane, 1,3-dimethoxypropane, pentafluoropropyl methyl ether, 2,2,3,3-tetrafluoropropyldifluoromethyl ether, tetrahydrofuran, and 2-methyltetrahydrofuran; esters such as methyl formate, methyl acetate, and γ-butyrolactone; nitriles such as acetonitrile and butyronitrile; amides such as N,N-dimethylformamide and N,N-dimethylacetamide; carbamates such as 3-methyl-2-oxazolidinone; and sulfur-containing compounds such as sulfolane, dimethyl sulfoxide, and 1,3-propane sultone can be used.

As the sodium-ion electrolyte, for example, an electrolyte prepared by dissolving the pre-embedding agent with the above-mentioned chemical structure of sodium organic sulfinate of the present disclosure in an organic solvent can be used. Examples of the electrolyte include the aforementioned carbonate electrolyte, sulfone electrolyte, ether electrolyte, and nitrile electrolyte, etc.

The electrolyte is selected according to the battery system. In the present disclosure, the preferred electrolyte is a carbonate electrolyte or an ether electrolyte. Calculated by the molar ratio (mol/L) of the lithium salt to the solvent volume in the electrolyte, the following combinations can be listed: 1.0 M $NaClO_4$ (EC:DEC=1:1), 1.0 M $LiClO_4$ (EC:EMC=3:7), 1.0 M $LiPF_6$ (EC:EMC=3:7) + 1% $LiClO_4$, 0.5 M LiBOB + 0.5 M LiTFSI (EC:EMC=3:7), 1.0 M $LiPF_6$ (DOL:DME=1:1) + 1% $LiClO_4$, etc.

[Pre-installed batteries and secondary batteries]

The pre-installed battery of the present disclosure is obtained by prepackaging the pre-embedded lithium/sodium electrode of the present disclosure. The pre-assembled battery is charged to form the battery and pre-embed lithium/sodium, and then package again to obtain the secondary battery of the present disclosure.

The present disclosure is not limited to the above-described examples, and various modifications can be made within the scope of the claims. Examples obtained by appropriately combining the technical means disclosed in different examples are also included in the technical scope of the present disclosure.

Examples

Although the present disclosure is described in detail based on the examples, the present disclosure is not limited thereto.

Example 1:

200 mmol of commercially available sodium trifluoromethanesulfinate was added to 500 ml of ethyl acetate and recrystallized. The obtained solid powder was placed in a vacuum oven and dried in vacuum at 60-70°C for 10 hours to obtain the final pre-embedded sodium material sodium trifluoromethanesulfinate. The pre-embedded sodium material is stable in dry air and can still be used normally after absorbing moisture and drying, and has good chemical stability.

100 mmol of sodium trifluoromethanesulfinate was added to 50 ml of acetonitrile, stirred evenly, and slowly added 10 ml of concentrated hydrochloric acid dropwise to form a white precipitate. After the addition was complete, the mixture was stirred at room temperature for 2 hours. After the reaction was complete, the precipitate was removed by filtration, and the precipitate was washed three times with ethyl acetate. The filtrate was collected, dried over anhydrous sodium sulfate, and the solvent was removed to obtain trifluoromethanesulfinic acid.

The obtained trifluoromethanesulfinic acid was dissolved in 80 ml of water, stirred evenly, and then 90 mmol of lithium hydroxide was added at 0°C. The temperature was slowly raised to room temperature and the mixture was reacted for 1 hour. After the reaction was complete, all the solvents were removed to obtain a white solid. An appropriate amount of ethyl acetate was added to the system to dissolve the solid. Dichloromethane was then added dropwise to the system until no more precipitation occured. Subsequently, the solid was obtained by filtration. The same method was used for recrystallization three times, and the obtained solid powder was placed in a vacuum oven and dried in vacuum at 60-70°C for 10 hours to obtain the final pre-embedded lithium material lithium trifluoromethanesulfinate. The pre-embedded lithium material is stable in dry air and can still be used normally after absorbing moisture and drying, and has good chemical stability.

Battery preparation and testing:

50 mg of sodium trifluoromethanesulfinate was added to 1 g of sodium battery electrolyte (1.0 mol $NaClO_4$ EC:DEC=1:1), stirred and dissolved to obtain a sodium battery electrolyte with a sodium pre-embedding function. Carbon paper was used as the positive electrode. In the glove box, the positive electrode case, carbon paper, separator, sodium sheet negative electrode, insulation gasket, wave spring and negative electrode case were placed in order, the electrolyte was added, and the coin cell was fixed and assembled using a battery tablet press. As shown in FIG. 1a, the assembled coin cell was tested for CV curve using an electrochemical workstation with a scan range of 2.5-4.0 V and a scan rate of 0.1 mV/s. It can be seen from the CV curve that sodium trifluoromethanesulfinate has irreversible desodiation capacity. As shown in FIG. 1b, the release capacity of sodium trifluoromethanesulfinate was tested using a coin cell test channel with a constant current of 0.06 mA for charging and discharging and a cut-off voltage of 2.5-4.0 V. From the charge/discharge curves, it can be seen that sodium trifluoromethanesulfinate had a capacity close to 170mAh/g, and the decomposition potential was between 3.6-3.7V, which had a suitable desodiation potential and met the basic requirements of pre-embedded sodium materials.

FIG. 1a is a CV curve test of the sodium trifluoromethanesulfinate coin cell in this Example. It can be seen that sodium trifluoromethanesulfinate began to charge and decompose at 3.7V, and sodium ions were released. FIG. 1b is the charge/discharge test of the sodium trifluoromethanesulfinate coin cell in this Example. It can be seen that sodium trifluoromethanesulfinate began to charge and decompose at 3.6V and had a capacity of nearly 170 mAh/g.

10 mg of lithium trifluoromethanesulfinate was added to 1 g of lithium battery electrolyte (1.0 mol $LiClO_4$ EC:EMC=3:7), stirred and dissolved to obtain a lithium battery electrolyte with a lithium pre-embedding function. Carbon paper was used as the positive electrode. In the glove box, the positive electrode case, carbon paper, separator, lithium negative electrode foil, insulation gasket, wave spring and negative electrode case were placed in order, the electrolyte was added, and the coin cell was fixed and assembled using a battery tablet press. As shown in FIG. 2a, the assembled coin cell was tested for CV curve using an electrochemical workstation with a scan range of 2.75-4.4V and a scan rate of 0.1 mV/s. It can be seen from the CV curve that lithium trifluoromethanesulfinate has irreversibledelithiation capacity. As shown in FIG. 2b, the release capacity of lithium trifluoromethanesulfinate was tested using a coin cell test channel with a constant current of 0.06mA for charging and discharging and a cut-off voltage of 2.75-4.3V. From the charge/discharge curves, it can be seen that lithium trifluoromethanesulfinate had a capacity close to 190mAh/g, and the decomposition potential was between 3.8-3.9V, which had a suitable delithiation potential and met the basic requirements of pre-embedded lithium materials.

FIG. 2a is a CV curve test of the lithium trifluoromethanesulfinate coin cell in this Example. It can be seen that lithium trifluoromethanesulfinate began to charge and decompose at 3.8V and lithium ions were released. FIG. 2b is the charge/discharge test of the lithium trifluoromethanesulfinate coin cell in this Example. It can be seen that lithium trifluoromethanesulfinate began to charge and decompose at 3.8 V and had a capacity of nearly 190 mAh/g.

FIG. 3 is an *in-situ* Raman characterization of the pre-embedded lithium method of lithium trifluoromethanesulfinate electrolyte of the example. It can be seen that the Raman characteristic peak of lithium trifluoromethanesulfinate gradually disappeared with the charging process, indicating that lithium trifluoromethanesulfinate in the electrolyte

gradually decomposed and released lithium ions. FIG. 3b is the Raman comparison of lithium trifluoromethanesulfinate powder, pure electrolyte, and lithium trifluoromethanesulfinate dissolved in pure electrolyte after charging in this Example. It can be seen that the Raman characteristic peak of lithium trifluoromethanesulfinate disappeared after charging. At this time, the characteristic peak of the electrolyte was basically consistent with the Raman characteristic peak of the pure electrolyte, indicating that lithium trifluoromethanesulfinate completely disappeared in the electrolyte after charging, and the charging lithium pre-embedding process had little effect on the electrolyte system.

FIG. 4 is a Raman comparison of a fresh sample of lithium trifluoromethanesulfinate and a sample stored in dry air in this Example. It can be seen that the Raman characteristic peaks of the fresh sample and the sample stored in dry air were basically consistent, indicating that the pre-embedded lithium material had good chemical stability.

Example 2:

200 mmol of commercially available sodium methanesulfinate was added to water and recrystallized. The obtained solid powder was placed in a vacuum oven and dried in vacuum at 60-70°C for 10 hours to obtain the final pre-embedded sodium material sodium methanesulfinate. The pre-embedded sodium material is stable in dry air and can still be used normally after absorbing moisture and drying, and has good chemical stability.

100 mmol of sodium methanesulfinate was added to 50 ml of acetonitrile, stirred evenly, and slowly added 10 ml of concentrated hydrochloric acid dropwise to form a white precipitate. After the addition was complete, the mixture was stirred at room temperature for 2 hours. After the reaction was complete, the precipitate was removed by filtration, and the filtrate was washed with ethyl acetate and precipitated three times. The filtrate was collected, dried over anhydrous sodium sulfate, and the solvent was removed to obtain methylsulfinic acid.

The obtained methylsulfinic acid was dissolved in 80 ml of water, stirred evenly, and then 90 mmol of lithium hydroxide was added at 0°C. The temperature was slowly raised to room temperature and the mixture was reacted for 1 hour. After the reaction was complete, all the solvents were removed to obtain a white solid, which was washed three times with ethanol. The obtained solid powder was placed in a vacuum oven and dried in vacuum at 60-70°C for 10 hours to obtain the final pre-embedded lithium material lithium methanesulfinate. The pre-embedded lithium material was stable in dry air and can still be used normally after absorbing moisture and drying, and had good chemical stability.

Battery preparation and testing:

100 mg of sodium methanesulfinate, 80 mg of Super P, 400 mg of PVDF solution (5% dissolved in NMP) and an appropriate amount of NMP were added to a ball mill and milled uniformly using agate beads. A 200 μm thick film was blade coated onto aluminum foil using a coating machine, and dried in vacuum at 60-70°C for 10 hours to obtain a sodium methanesulfinate electrode sheet, which was then cut into Φ12 discs. In the glove box, the positive electrode case, sodium methanesulfinate electrode sheet, separator, sodium sheet negative electrode, insulation gasket, wave spring and negative electrode case were placed in order, fixed using a battery tablet press, and assembled into a coin cell. As shown in FIG. 5a, the release capacity of sodium methanesulfinate was tested using a coin cell test channel with a constant current of 0.06mA for charging and discharging, and a cut-off voltage of 2.5-4.0 V. From the charge/discharge curves, it can be seen that sodium methanesulfinate had a capacity close to 262 mAh/g, and the decomposition potential was between 3.2-3.3V, which had a suitable desodiation potential and met the basic requirements of pre-embedded sodium materials.

FIG. 5a is the charge/discharge test of the sodium methanesulfinate coin cell in this Example. It can be seen that sodium methanesulfinate began charge and decompose at 3.2V and has a capacity of nearly 262 mAh/g. This indicates that the pre-embedded sodium material had a suitable desodiation potential and met the basic requirements of pre-embedded sodium materials.

100 mg of lithium methanesulfinate, 80 mg of Super P, 400 mg of PVDF solution (5% dissolved in NMP) and an appropriate amount of NMP were added to a ball mill and milled uniformly using agate beads. A 200 μm thick film was blade coated onto aluminum foil using a coating machine, and dried in vacuum at 60-70°C for 10 hours to obtain a lithium methanesulfinate electrode sheet, which was then cut into Φ12 discs. In the glove box, the positive electrode case, lithium methanesulfinate electrode sheet, separator, lithium sheet negative electrode, insulation gasket, wave spring and negative electrode case were placed in order, fixed using a battery tablet press, and assembled into a coin cell. As shown in FIG. 5b, the release capacity of lithium methanesulfinate was tested using a coin cell test channel with a constant current of 0.06mA for charging and discharging, and a cut-off voltage of 2.75-4.3V. From the charge/discharge curves, it can be seen that lithium methanesulfinate had a capacity close to 310 mAh/g, and the decomposition potential was between 3.7-3.8V, which had a suitable delithiation potential and met the basic requirements of pre-embedded lithium materials.

FIG. 5 is the charge/discharge test of the lithium methanesulfinate coin cell in this Example. It can be seen that lithium methanesulfinate begins to charge and decompose at 3.7V and has a capacity of nearly 311 mAh/g. This indicates that the pre-lithium embedded material has a suitable delithiation potential and meets the basic requirements of pre-lithium

embedded materials.

Example 3:

200 mmol of commercially available sodium ethylsulfinate was added to water and recrystallized. The obtained solid powder was placed in a vacuum oven and dried in vacuum at 60-70 °C for 10 hours to obtain the final pre-embedded sodium material sodium ethylsulfinate. The pre-embedded sodium material is stable in dry air and can still be used normally after absorbing moisture and drying, and has good chemical stability.

100 mmol of sodium ethylsulfinate was added to 50 ml of acetonitrile, stirred evenly, and slowly added 10 ml of concentrated hydrochloric acid dropwise to form a white precipitate. After the addition was complete, the mixture was stirred at room temperature for 2 hours. After the reaction was complete, the precipitate was removed by filtration, and the filtrate was washed with ethyl acetate and precipitated three times. The filtrate was collected, dried over anhydrous sodium sulfate, and the solvent was removed to obtain ethylsulfinic acid.

The obtained ethylsulfinic acid was dissolved in 80 ml of water, stirred evenly, and then 90 mmol of lithium hydroxide was added at 0°C. The temperature was slowly raised to room temperature and the mixture was reacted for 1 hour. After the reaction was complete, all the solvents were removed to obtain a white solid, which was washed three times with ethanol. The obtained solid powder was placed in a vacuum oven and dried in vacuum at 60-70°C for 10 hours to obtain the final pre-embedded lithium material lithium ethylsulfinate. The pre-embedded lithium material was stable in dry air and can still be used normally after absorbing moisture and drying, and had good chemical stability.

Battery preparation and testing:

100 mg of sodium ethylsulfinate, 80 mg of Super P, 400 mg of PVDF solution (5% dissolved in NMP) and an appropriate amount of NMP were added to a ball mill and milled uniformly using agate beads. A 200 μm thick film was blade coated onto aluminum foil using a coating machine, and vacuum dried in vacuum at 60-70°C for 10 hours to obtain a sodium ethylsulfinate electrode sheet, which was then cut into Φ12 discs. In the glove box, the positive electrode case, sodium ethylsulfinate electrode sheet, separator, sodium sheet negative electrode, insulation gasket, wave spring and negative electrode case were placed in order, fixed using a battery tablet press, and assembled into a coin cell. As shown in FIG. 5a, the release capacity of sodium ethylsulfinate was tested using a coin cell test channel with a constant current of 0.06mA for charging and discharging, and a cut-off voltage of 2.5-4.0 V. From the charge/discharge curves, it can be seen that sodium ethylsulfinate had a capacity close to 230 mAh/g, and the decomposition potential was between 3.1-3.2V, which had a suitable desodiation potential and met the basic requirements of pre-embedded sodium materials.

FIG. 5a is the charge/discharge test of the sodium ethylsulfinate coin cell in this Example. It can be seen that sodium ethylsulfinate begins to charge and decompose at 3.1V and has a capacity of nearly 230 mAh/g. This indicates that the pre-embedded sodium material has a suitable desodiation potential and meets the basic requirements of pre-embedded sodium materials.

100 mg of lithium ethylsulfinate, 80 mg of Super P, 400 mg of PVDF solution (5% dissolved in NMP) and an appropriate amount of NMP were added to a ball mill and milled uniformly using agate beads. A 200 μm thick film was blade coated onto aluminum foil using a coating machine, and vacuum dried in vacuum at 60-70°C for 10 hours to obtain a lithium ethylsulfinate electrode sheet, which was then cut into Φ12 discs. In the glove box, the positive electrode case, lithium ethylsulfinate electrode sheet, separator, lithium sheet negative electrode, insulation gasket, wave spring and negative electrode case were placed in order, fixed using a battery tablet press, and assembled into a coin cell. As shown in FIG. 5b, the release capacity of lithium ethylsulfinate was tested using a coin cell test channel, with a constant current of 0.06mA for charging and discharging, and a cut-off voltage of 2.75-4.3V. From the charge/discharge curves, it can be seen that lithium ethylsulfinate had a capacity close to 265 mAh/g, and the decomposition potential was between 3.8-3.9V, which had a suitabledelithiation potential and met the basic requirements of pre-embedded lithium materials.

FIG. 5b is the charge/discharge test of the lithium ethylsulfinate coin cell in this Example. It can be seen that lithium ethylsulfinate began to charge and decompose at 3.8V and has a capacity of nearly 265 mAh/g. This indicates that the pre-lithium embedded material had a suitable delithiation potential and met the basic requirements of pre-lithium embedded materials.

Example 4:

200 mmol of commercially available sodium phenylsulfinate was added to water and recrystallized. The obtained solid powder was placed in a vacuum oven and dried in vacuum at 60-70°C for 10 hours to obtain the final pre-embedded sodium material sodium phenylsulfinate. The pre-embedded sodium material is stable in dry air and can still be used normally after absorbing moisture and drying, and has good chemical stability.

100 mmol of sodium phenylsulfinate was added to 50 ml of acetonitrile, stirred evenly, and slowly added 10 ml of

concentrated hydrochloric acid dropwise to form a white precipitate. After the addition was complete, the mixture was stirred at room temperature for 2 hours. After the reaction was complete, the precipitate was removed by filtration, and the filtrate was washed with ethyl acetate and precipitated three times. The filtrate was collected, dried over anhydrous sodium sulfate, and the solvent was removed to obtain phenylsulfinic acid.

The obtained phenylsulfinic acid was dissolved in 80 ml of water, stirred evenly, and then 90 mmol of lithium hydroxide was added at 0°C. The temperature was slowly raised to room temperature and the mixture was reacted for 1 hour. After the reaction was completed, all the solvents were removed to obtain a white solid, which was washed three times with ethanol. The obtained solid powder was placed in a vacuum oven and dried in vacuum at 60-70°C for 10 hours to obtain the final pre-embedded lithium material lithium phenylsulfinate. The pre-embedded lithium material was stable in dry air and can still be used normally after absorbing moisture and drying, and had good chemical stability.

Battery preparation and testing:

100 mg of sodium phenylsulfinate, 80 mg of Super P, 400 mg of PVDF solution (5% dissolved in NMP) and an appropriate amount of NMP were added to a ball mill and milled uniformly using agate beads. A 200 μm thick film was blade coated onto aluminum foil using a coating machine, and vacuum dried in vacuum at 60-70°C for 10 hours to obtain a sodium phenylsulfinate electrode sheet, which was then cut into Φ12 discs. In the glove box, the positive electrode case, sodium phenylsulfinate electrode sheet, separator, sodium sheet negative electrode, insulation gasket, wave spring and negative electrode case were placed in order, fixed using a battery tablet press, and assembled into a coin cell. As shown in FIG. 5a, the release capacity of sodium phenylsulfinate was tested using a coin cell test channel with a constant current of 0.06mA for charging and discharging, and a cut-off voltage of 2.5-4.0 V. From the charge/discharge curves, it can be seen that sodium phenylsulfinate had a capacity close to 163 mAh/g, and the decomposition potential was between 3.3-3.4V, which had a suitable desodiation potential and met the basic requirements of pre-embedded sodium materials.

FIG. 5a is the charge/discharge test of the sodium phenylsulfinate coin cell in this Example. It can be seen that sodium phenylsulfinate begins to charge and decompose at 3.3V and has a capacity of nearly 163 mAh/g. This indicates that the pre-embedded sodium material has a suitable desodiation potential and meets the basic requirements of pre-embedded sodium materials.

100 mg of lithium phenylsulfinate, 80 mg of Super P, 400 mg of PVDF solution (5% dissolved in NMP) and an appropriate amount of NMP were added to a ball mill and milled uniformly using agate beads. A 200 μm thick film was blade coated onto aluminum foil using a coating machine, and vacuum dried in vacuum at 60-70°C for 10 hours to obtain a lithium phenylsulfinate electrode sheet, which was then cut into Φ12 discs. In the glove box, the positive electrode case, lithium phenylsulfinate electrode sheet, separator, lithium sheet negative electrode, insulation gasket, wave spring and negative electrode case were placed in order, fixed using a battery tablet press, and assembled into a coin cell. As shown in FIG. 5b, the release capacity of lithium phenylsulfinate was tested using a coin cell test channel with a constant current of 0.06mA for charging and discharging, and a cut-off voltage of 2.75-4.3V. From the charge/discharge curves, it can be seen that lithium phenylsulfinate had a capacity close to 180 mAh/g, and the decomposition potential was between 3.9-4.0V, which had a suitable delithiation potential and met the basic requirements of pre-embedded lithium materials.

FIG. 5b is the charge/discharge test of the lithium phenylsulfinate coin cell in this Example. It can be seen that lithium phenylsulfinate began to charge and decompose at 3.9V and had a capacity of nearly 181 mAh/g. This indicates that the pre-lithium embedded material had a suitable delithiation potential and met the basic requirements of pre-lithium embedded materials.

Example 5:

200 mmol of commercially available sodium p-tolylsulfinate was added to water and recrystallized. The obtained solid powder was placed in a vacuum oven and dried in vacuum at 60-70°C for 10 hours to obtain the final pre-embedded sodium material sodium p-tolylsulfinate. The pre-embedded sodium material is stable in dry air and can still be used normally after absorbing moisture and drying, and has good chemical stability.

100 mmol of sodium p-tolylsulfinate was added to 50 ml of acetonitrile, stirred evenly, and slowly added 10 ml of concentrated hydrochloric acid dropwise to form a white precipitate. After the addition was complete, the mixture was stirred at room temperature for 2 hours. After the reaction was complete, the precipitate was separated by filtration, and the filtrate was washed with ethyl acetate and precipitated three times. The filtrate was collected, dried over anhydrous sodium sulfate, and the solvent was removed to obtain p-tolylsulfinic acid.

The obtained p-tolylsulfinic acid was dissolved in 80 ml of water, stirred evenly, and then 90 mmol of lithium hydroxide was added at 0°C. The temperature was slowly raised to room temperature and the mixture was reacted for 1 hour. After the reaction was complete, all the solvents were removed to obtain a white solid, which was washed three times with ethanol. The obtained solid powder was placed in a vacuum oven and dried in vacuum at 60-70°C for 10 hours to obtain the final pre-embedded lithium material lithium p-tolylsulfinate. The pre-embedded lithium material was stable in dry

air and can still be used normally after absorbing moisture and drying, and had good chemical stability.

Battery preparation and testing:

100 mg of sodium p-tolylsulfinate, 80 mg of Super P, 400 mg of PVDF solution (5% dissolved in NMP) and an appropriate amount of NMP were added to a ball mill and milled uniformly using agate beads. A 200 μm thick film was blade coated onto aluminum foil using a coating machine, and vacuum dried in vacuum at 60-70°C for 10 hours to obtain a sodium p-tolylsulfinate electrode sheet, which was then cut into Φ12 discs. In the glove box, the positive electrode case, sodium p-toluenesulfinate electrode sheet, separator, sodium sheet negative electrode, insulation gasket, wave spring and negative electrode case were placed in order, fixed using a battery tablet press, and assembled into a coin cell. As shown in FIG. 5a, the release capacity of sodium phenylsulfinate was tested using a coin cell test channelwith a constant current of 0.06mA for charging and discharging, and a cut-off voltage of 2.5-4.0 V. From the charge/discharge curves, it can be seen that sodium p-tolylsulfinate had a capacity close to 150 mAh/g, and the decomposition potential was between 3.3-3.4V, which had a suitable desodiation potential and met the basic requirements of pre-embedded sodium materials.

FIG. 5a is the charge/discharge test of the sodium p-tolylsulfinate coin cell in this Example. It can be seen that sodium p-tolylsulfinate begins to charge and decompose at 3.3V and has a capacity of nearly 150 mAh/g. This indicates that the pre-embedded sodium material has a suitable desodiation potential and meets the basic requirements of pre-embedded sodium materials.

100 mg of lithium p-tolylsulfinate, 80 mg of Super P, 400 mg of PVDF solution (5% dissolved in NMP) and an appropriate amount of NMP were added to a ball mill and milled uniformly using agate beads. A 200 μm thick film was blade coated onto aluminum foil using a coating machine, and vacuum dried in vacuum at 60-70°C for 10 hours to obtain a lithium p-tolylsulfinate electrode sheet, which was then cut into Φ12 discs. In the glove box, the positive electrode case, lithium p-toluenesulfinate electrode sheet, separator, lithium sheet negative electrode, insulation gasket, wave spring and negative electrode case were placed in order, fixed using a battery tablet press, and assembled into a coin cell. As shown in FIG. 5b, the release capacity of lithium p-tolylsulfinate was tested using a coin cell test channel with a constant current of 0.06mA for charging and discharging, and a cut-off voltage of 2.75-4.3V. From the charge/discharge curves, it can be seen that lithium p-tolylsulfinate had a capacity close to 165 mAh/g, and the decomposition potential was between 3.9-4.0V, which had a suitabledelithiation potential and met the basic requirements of pre-embedded lithium materials.

FIG. 5b is the charge/discharge test of the lithium p-tolylsulfinate coin cell in this Example. It can be seen that lithium p-tolylsulfinate begins to charge and decompose at 3.9V and has a capacity of nearly 165 mAh/g. This indicates that the pre-lithium embedded material has a suitable delithiation potential and meets the basic requirements of pre-lithium embedded materials.

Example 6:

Commercially available sodium p-chlorophenylsulfinate was used as pre-embedded sodium material instead of sodium methanesulfinate in Example 2. In addition, the pre-embedded lithium material lithium p-chlorophenylsulfinate was prepared in the same manner as in Example 2 using sodium p-chlorophenylsulfinate. The pre-embedded lithium material is stable in dry air and can still be used normally after absorbing moisture and drying, and has good chemical stability.

Battery preparation and testing:

A battery was prepared and tested in the same manner as in Example 2. The desorption capacity of sodium p-chlorophenylsulfinate was tested using a coin cell test channel. Constant current charge/discharge was used, with a current of 0.06 mA and a cut-off voltage of 2.5-4.0 V. It was measured that the sodium p-chlorophenylsulfinate had a capacity of close to 135 mAh/g, and the decomposition potential was between 3.3-3.4V, which had a suitable desodiation potential and met the basic requirements of pre-embedded sodium materials. Similarly, the desorption capacity of lithium p-chlorophenylsulfinate was tested using a coin cell test channel. Constant current charge/discharge was used, with a current of 0.06 mA and a cut-off voltage of 2.75-4.3V. It was measured that the lithium p-chlorophenylsulfinate had a capacity of close to 147 mAh/g, and the decomposition potential was between 3.9-4.0V, which had a suitable delithiation potential and met the basic requirements of pre-embedded lithium materials.

Example 7:

Commercially available sodium tetrahydropyran-4-sulfinate was used as pre-embedded sodium material instead of sodium methanesulfinate in Example 2. In addition, the pre-embedded lithium material lithium tetrahydropyran-4-sulfinate was prepared in the same manner as in Example 2 using sodium tetrahydropyran-4-sulfinate. The pre-embedded lithium material is stable in dry air and can still be used normally after absorbing moisture and drying, and has good chemical stability.

Battery preparation and testing:

A battery was prepared and tested in the same manner as in Example 2. The desorption capacity of sodium tetrahydropyran-4-sulfinate was tested using a coin cell test channel. Constant current charge/discharge was used, with a current of 0.06 mA and a cut-off voltage of 2.5-4.0 V. It was measured that the sodium tetrahydropyran-4-sulfinate had a capacity of close to 157 mAh/g, and the decomposition potential was between 3.3-3.4V, which had a suitable desodiation potential and met the basic requirements of pre-embedded sodium materials. Similarly, the desorption capacity of lithium tetrahydropyran-4-sulfinate was tested using a coin cell test channel. Constant current charge/discharge was used, with a current of 0.06 mA and a cut-off voltage of 2.75-4.3V. It was measured that the lithium tetrahydropyran-4-sulfinate had a capacity of close to 173 mAh/g, and the decomposition potential was between 3.9-4.0V, which had a suitable delithiation potential and met the basic requirements of pre-embedded lithium materials.

Example 8:

A lithium-free pouch cell with a capacity of approximately 28.6 Ah was constructed by using a lithium-free positive electrode material sulfurized polyacrylonitrile SPAN combined with a graphite system, and dissolving 2 wt% lithium trifluoromethanesulfinate in the electrolyte.

The graphite was commercially available (Sigma-Aldrich).The elemental analysis results of the sulfurized polyacrylonitrile showed a sulfur content of 39.5%, a carbon content of 40.58%, a nitrogen content of 15.05%, and a hydrogen content of 0.59%.

Conductive carbon, polyacrylonitrile sulfide, and PVDF in a ratio of 90:5:5 and an appropriate amount of NMP were mixed and thoroughly stirred. The mixture was evenly blade coated onto aluminum foil, dried, and cut to obtain a positive electrode sheet. Graphite, PVDF, and NMP in a ratio of 92:4:4 and an appropriate amount of NMP were mixed and stirred thoroughly. The mixture was evenly blade coated onto the copper foil, dried, and cut to obtain the negative electrode sheet. A lithium-free pouch cell of approximately 28.6 Ah were constructed with multilayer stacking method, and the lithium compensation of battery system was achieved through charging.

As shown in FIG. 7, its energy density reached 360 Wh $kg^{-1}$. Its initial coulombic efficiency reached 99.7%, and after 350 cycles of charge/discharge, its capacity retention rate was still above 80%.

Example 9:

A lithium pouch cell with a capacity of approximately 14 Ah was constructed by using a positive electrode material NCM811 ($LiNi_{0.8}Mn_{0.1}Co_{0.1}O_2$) combined with a SiO system and dissolving 2 wt% lithium trifluoromethanesulfinate in the electrolyte.

SiO, conductive carbon (Super P), polyacrylic acid (PAA) and carbon nanotubes (CNT) in a ratio of 80:10:4:6 and an appropriate amount of NMP were mixed and stirred evenly. The mixture was coated on copper foil dried, and cut to obtain a positive electrode sheet. NCM 811, Super P, and PVDF in a ratio of 92:4:4 and an appropriate amount of NMP were mixed and stirred evenly. Then the mixture was coated on aluminum foil , dried and cut to obtain a negative electrode. In a capacity ratio of the negative electrode to the positive electrode capacity of 1.01, 14 Ah dry cell was assembled and lithium compensation of the battery system was achieved through charging.

As shown in FIG. 8, its energy density reached 352 Wh $kg^{-1}$, its initial coulombic efficiency reached 99.7%, and its capacity retention rate was still above 87.6% after 800 cycles of charge/discharge.

Example 10:

A lithium pouch cell with a capacity of approximately 3.0 Ah was constructed by using a positive electrode material NCM811 ($LiNi_{0.8}Mn_{0.1}Co_{0.1}O_2$) combined with a SiO system and dissolving 2 wt% lithium trifluoromethanesulfinate in the electrolyte.

The test conditions were as follows: The amount of the loaded electrode material was 4.0 mAh cm-2. The surface capacity ratio of negative electrode to positive electrode was 1.05. and charge/discharge test was at 0.5 C rate. As shown in FIG. 9, when no lithium compensation material was added, the initial efficiency of the pouch cell was 57%. When commercial lithium compensation agent LNC was added, its initial effect was increased to 62% at its maximum addition amount of 4% by mass. As shown in FIG. 9, by lithium compensation through the electrolyte in the present disclosure, the initial efficiency can be increased to 100%. Compared with the battery system without adding lithium compensation materials and adding commercial lithium compensation agent LNC, cycle stability of the battery was not affected while

effectively improving the initial efficiency of the battery while the initial efficiency of the battery was effectively improved. After 800 stable cycles, its capacity retention rate was above 90%. It indicated that the present disclosure had good practical applicability for lithium-poor battery systems.

Example 11:

Sodium trifluoromethanesulfinate was dissolved in the electrolyte at 5 wt% to obtain a sodium electrolyte with sodium pre-embedding function. A sodium dry cell was assembled and injected with sodium electrolyte, and charged to achieve sodium compensation in the battery system. As shown in FIG. 10, in a sodium positive electrode to copper battery system, after sodium trifluoromethanesulfinate was added to the electrolyte, its charge/discharge capacity was significantly improved.

Example 12:

Sodium methanesulfinate was mixed at a ratio of 90 wt% with a mixture of certain ratio of conductive carbon and binder to obtain a sodium compensation slurry. The slurry is evenly blade coated on the positive electrode sheet with a certain thickness and dried. Then a sodium dry cell was assembled, injected with sodium electrolyte, and charged to achieve sodium compensation in the battery system. As shown in FIG. 11, in a sodium positive electrode to copper battery system, after a coating of sodium methanesulfinate is applied on the positive electrode surface by blade coating, its charge/discharge capacity was significantly improved.

Example 13:

Sodium methanesulfinate was mixed at a ratio of 5 wt% with a mixture of certain ratio of sodium positive electrode material, conductive carbon and binder to obtain a sodium positive electrode slurry. The slurry is evenly blade coated on the current collector with a certain thickness and dried. Then a sodium dry cell was assembled , injected with sodium electrolyte, and charged to achieve sodium compensation in the battery system. As shown in FIG. 12, in a sodium positive electrode to copper battery system, after a certain ratio of sodium methanesulfinate was mixed in the positive electrode, its charge/discharge capacity was significantly improved.

Example 14:

A sodium-poor P2-type layered oxide ($Na_{0.67}Mn_{0.67}Ni_{0.33}O_2$) and hard carbon system were used to construct a sodium pouch cell with a capacity of approximately 1 Ah,where 5 wt% sodium trifluoromethanesulfinate was dissolved in the electrolyte.

$Na_{0.67}Mn_{0.67}Ni_{0.33}O_2$, conductive carbon (Super P) and (PVDF) in a ratio of 80:10:10 with an appropriate amount of NMP were stirred evenly, and the mixture was coated on aluminum foil, dried and cut to prepare a positive electrode sheet. Hard carbon, Super P, and PVDF in a ratio of 92:4:4 with an appropriate amount of NMP were stirred evenly, and the mixture was coated on copper foil, dried, cut to prepare a negative electrode. Then they were assembled into dry cell. In a capacity ratio of the negative electrode to the positive electrode of 1.1, 1 Ah dry cell was assembled and sodium compensation of the battery system was achieved through charging.

As shown in FIG. 13, without sodium compensation, the full cell has an initial capacity of only 77 mAh/g. By sodium compensation with the electrolyte, an initial capacity of 96 mAh/g was achieved, which reached the level of industrial sodium-rich positive electrode full cells.

Example 15:

A lithium pouch cell with a capacity of approximately 1 Ah was constructed using a system of graphite negative electrode and a lithium iron phosphate positive electrode.

Conductive carbon, lithium iron phosphate, and PVDF in a ratio of 90:5:5 and an appropriate amount of NMP were mixed and thoroughly stirred. The mixture was evenly blade coated onto aluminum foil, dried, and cut to obtain a positive electrode sheet. Graphite, PVDF, and NMP in a ratio of 92:4:4 and an appropriate amount of NMP were mixed and stirred thoroughly. The mixture was evenly blade coated onto the copper foil, dried, and cut to obtain the negative electrode sheet. 1 Ah pouch cell were constructed with multilayer stacking method. After 1800 cycles of 1 C charge/discharge, the capacity decayed to 85%, resulting in a waste battery.

Without destroying the overall structure of the waste battery, the electrolyte in the battery was discharged from the battery. 2 wt% lithium trifluoromethanesulfinate was dissolved in the electrolyte, and the electrolyte containing the lithium compensation agent was injected into the waste battery, and the lithium compensation of the battery system was

performed through charging.

As shown in FIG. 14, after about 1800 cycles, the battery system capacity dropped to 85%. By using the method of lithium compensation through the electrolyte described in the present disclosure, its capacity can be increased again to 99.6%. And after about 1800 cycles, the battery system capacity dropped to 85% again. By using the method of lithium compensation through the electrolyte described in the present disclosure, its capacity can be increased again to 98.8%. And through the third cycle and electrolyte lithium compensation, the battery capacity can be restored to 98.1% again. After 7717 cycles, the battery capacity retention rate reaches 97.4%. According to trend forecasts, 30,000-60,000 battery cycles can be achieved.

The initial coulombic efficiency test method: Under constant current charge/discharge test conditions, the ratio of the initial discharge capacity to the initial charge capacity is the initial coulombic efficiency.

Method for determining the capacity retention rate after n cycles of charge/discharge: Under constant current charge/discharge test conditions, the ratio of the discharge capacity of the nth cycle to the discharge capacity of the first cycle is the capacity retention rate after n cycles of charge/discharge.

Energy density test method: The product of the total capacity of the battery and the discharge voltage platform value is the battery capacity, the ratio of the battery capacity to the battery mass is the battery weight energy density, and the ratio of the battery capacity to the battery mass is the battery volume energy density.

The residual pre-embedding agent content in the electrodes of the above examples was measured and calculated, and it was found that they were all below 0.005 mmol.

## Industrial applicability

According to the present disclosure, a pre-embedding agent, a pre-embedded lithium/sodium positive electrode, a lithium/sodium ion battery containing the same, and a lithium/sodium pre-embedding method are provided, which remains no by-products in the battery after pre-embedding lithium/sodium, has good stability, a safe and convenient pre-embedding lithium/sodium process, and a high energy density and capacity retention rate.

## Claims

1. A pre-embedding agent for pre-embedding lithium or pre-embedding sodium, **characterized in that**, comprising an organic sulfinate represented by following formula (1) as a framework,

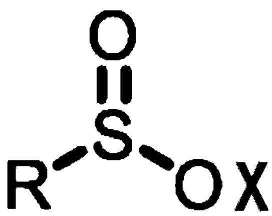

formula (1)

in the formula (1), R is selected from an alkyl group or a halogenated alkyl group having 1 to 10 carbon atoms, an aryl group or a halogenated aryl group having a benzene ring, a thiophene ring, a furan ring, or a pyridine ring, or a chain ether group or a cyclic ether group having 1 to 10 carbon atoms, a chain ester group or a cyclic ester group having 1 to 10 carbon atoms, and X represents Na or Li.

2. The pre-embedding agent according to claim 1, **characterized in that**, in the formula (1), R is the alkyl group selected from -Me, or -Et, the halogenated alkyl group selected from $-CF_3$, $-C_2F_5$, or $-C_4F_9$, the aryl group selected from -Ph, $-CH_3C_6H_4$, $-C_5NH_4$, $-C_4H_3S$, or $-C_4H_3O$; or the halogenated aryl group selected from $-FC_6H_4$, or $-ClC_6H_4$.

3. A pre-embedded lithium positive electrode, **characterized in that**, comprising a current collector where a structure of an active material layer containing a positive electrode active material, a conductive agent and a binder is formed, wherein a lithium pre-embedding treatment is performed using the pre-embedding agent according to claim 1 or 2, X in the formula (1) represents Li;

   based on a battery capacity of 1Ah, a content of the pre-embedding agent in the pre-embedded lithium positive electrode before the lithium pre-embedding treatment is n mol, and the content of the pre-embedding agent in the

pre-embedded lithium positive electrode after the lithium pre-embedding treatment is less than 0.005 mmol,

$$n = \frac{c \cdot 3.6}{F},$$

in the above formula, F is Faraday constant, and c is an amount of pre-embedded lithium measured in Ah for the battery capacity of 1Ah.

4. The pre-embedded lithium positive electrode according to claim 3, **characterized in that**, the positive electrode active material is selected from lithium iron phosphate, sulfurized polyacrylonitrile (SPAN), ternary materials, lithium cobalt oxide, lithium manganese oxide, titanium disulfide, vanadium pentoxide, or Prussian blue.

5. A pre-installed lithium-ion secondary battery, **characterized in that**, comprising the pre-embedded lithium positive electrode according to claim 3 or 4, and a negative electrode.

6. The pre-installed lithium-ion secondary battery according to claim 5, **characterized in that**, the negative electrode is selected from graphite, lithium metal, silicon-carbon, silicon, a composite of silicon and graphite, a composite of silicon and lithium metal, a composite of graphite and lithium metal, silicon monoxide, or black phosphorus.

7. A lithium-ion secondary battery, **characterized in that**, obtained by performing the lithium pre-embedding treatment to the pre-installed lithium-ion secondary battery according to claim 5 or 6.

8. A pre-embedded sodium positive electrode, comprising a current collector where a structure of an active material layer containing a positive electrode active material, a conductive agent and a binder is formed, **characterized in that**, a sodium pre-embedding treatment is performed using the pre-embedding agent according to claim 1 or 2, X in the formula (1) represents Na;

based on a battery capacity of 1Ah, a content of the pre-embedding agent in the pre-embedded sodium positive electrode before the sodium pre-embedding treatment is n mol, and the content of the pre-embedding agent in the pre-embedded sodium positive electrode after the sodium pre-embedding treatment is less than 0.005 mmol,

$$n = \frac{c \cdot 3.6}{F},$$

in the above formula, F is Faraday constant, and c is an amount of pre-embedded sodium measured in Ah for the battery capacity of 1Ah.

9. The pre-embedded sodium positive electrode according to claim 8, **characterized in that**, the positive electrode active material is selected from P2-type layered oxide whose chemical formula is $Na_{0.67}Mn_{0.67}Ni_{0.33}O_2$, Prussian blue, O3-type layered oxide whose chemical formula is $NaNi_{0.33}Fe_{0.33}Mn_{0.33}O_2$, or sodium vanadium phosphate.

10. A pre-installed sodium-ion secondary battery, **characterized in that**, comprising the pre-embedded sodium positive electrode according to claim 8 or 9.

11. The pre-installed sodium-ion secondary battery according to claim 10, **characterized in that**, a negative electrode is selected from soft carbon or hard carbon.

12. A sodium-ion secondary battery, **characterized in that**, obtained by performing the sodium pre-embedding treatment to the pre-installed sodium-ion secondary battery according to claim 10 or 11.

13. A method for pre-embedding lithium or pre-embedding sodium, **characterized in that**, comprising performing a lithium pre-embedding treatment or a sodium pre-embedding treatment using the pre-embedding agent according to claim 1 or 2.

14. The method for pre-embedding lithium or pre-embedding sodium according to claim 13, **characterized in that**, perform the lithium pre-embedding treatment or sodium pre-embedding treatment comprises dissolving the pre-embedding agent in an electrolyte.

**15.** The method for pre-embedding lithium or pre-embedding sodium according to claim 14, **characterized in that**, further comprising, for a used battery electrode, dissolving the pre-embedding agent in an electrolyte, and performing the lithium pre-embedding treatment or sodium pre-embedding treatment comprises performing the lithium pre-embedding treatment or the sodium pre-embedding treatment by adding the electrolyte.

**16.** The method for pre-embedding of lithium or pre-embedding sodium according to claim 14 or 15, **characterized in that**, dissolving the pre-embedding agent in the electrolyte comprises dissolving the pre-embedding agent in the electrolyte at a mass fraction of 0.1% to 30%.

**17.** The method for pre-embedding lithium or pre-embedding sodium according to claim 13, **characterized in that**, performing the lithium pre-embedding treatment or the sodium pre-embedding treatment comprises doping the pre-embedding agent into a positive electrode.

**18.** The method for pre-embedding lithium or pre-embedding sodium according to claim 13, **characterized in that**, performing the lithium pre-embedding treatment or the sodium pre-embedding treatment comprises coating the pre-embedding agent on a positive electrode.

**19.** The method for pre-embedding lithium or pre-embedding sodium according to any one of claims 13 to 15, 17, and 18, **characterized in that**, the electrolyte is a carbonate ester electrolyte or an ether electrolyte.

**20.** The method for pre-embedding lithium or pre-embedding sodium according to claim 19, **characterized in that**, the carbonate ester electrolyte is selected from 1.0 mol $NaClO_4$, 1.0 mol $LiClO_4$, 1.0 mol $LiPF_6$ + 1% $LiClO_4$, 0.5 mol LiBOB + 0.5 mol LiTFSI, or the ether electrolyte is 1.0 mol $LiPF_6$ + 1% $LiClO_4$.

**21.** A production method of a pre-embedding agent for pre-embedding lithium, **characterized in that**, comprising following steps:

Step (1): weighing a sodium organic sulfinate sodium salt whose chemical formula is in accordance with following formula (1) in claim 1 or 2 with X represents Na, placing the sodium organic sulfinate sodium salt in a solvent, and recrystallizing to obtain a pre-embedding sodium material;
Step (2): weighing the pre-embedding sodium material in the step (1), placing the pre-embedding sodium material in an organic solvent, dropwise adding an appropriate amount of acid, stirring to react, extracting and drying, and evaporating the organic solvent to obtain a product;
Step (3): placing the product obtained in the step (2) in deionized water, adding an appropriate amount of lithium salt, stirring to react, and evaporating the solvent to obtain a solid; and
Step (4): recrystallizing the solid obtained in the step (3) using ethyl acetate and dichloromethane to obtain a solid power, drying the solid powder to obtain an lithium organic sulfinate salt whose chemical formula is in accordance with the formula (1) in claim 1 or 2 with X represents Li.

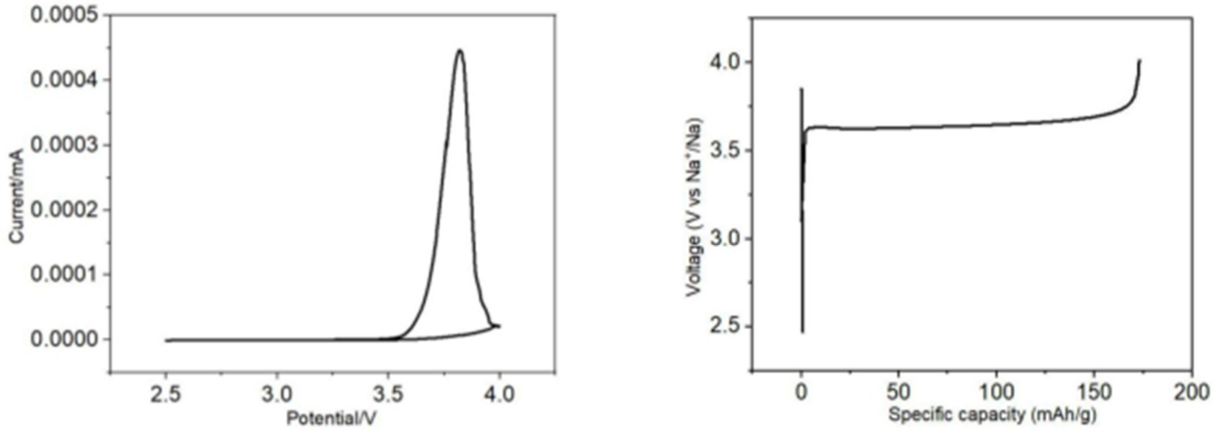


a b

FIG.1

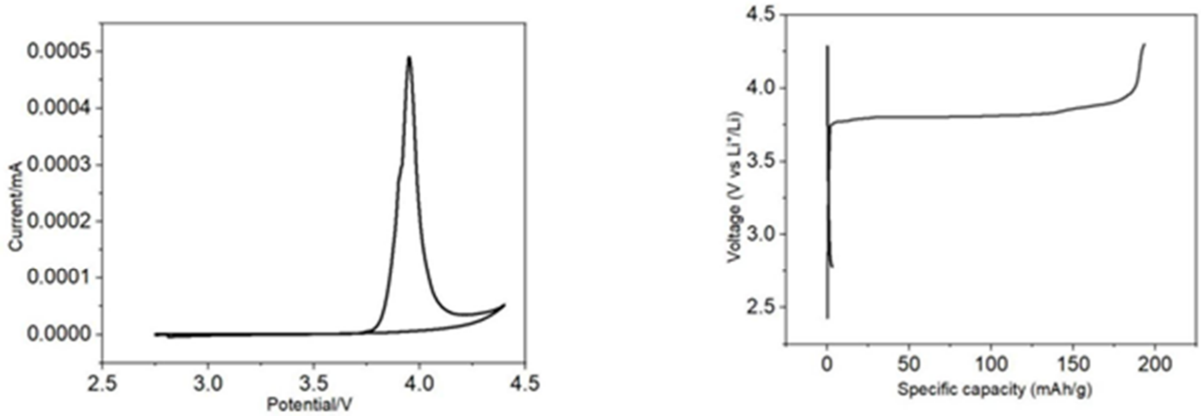


a b

FIG.2

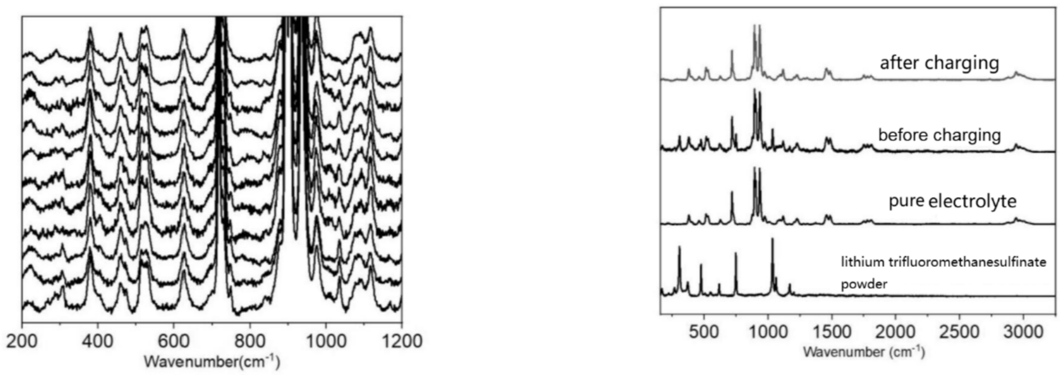


a b

FIG.3

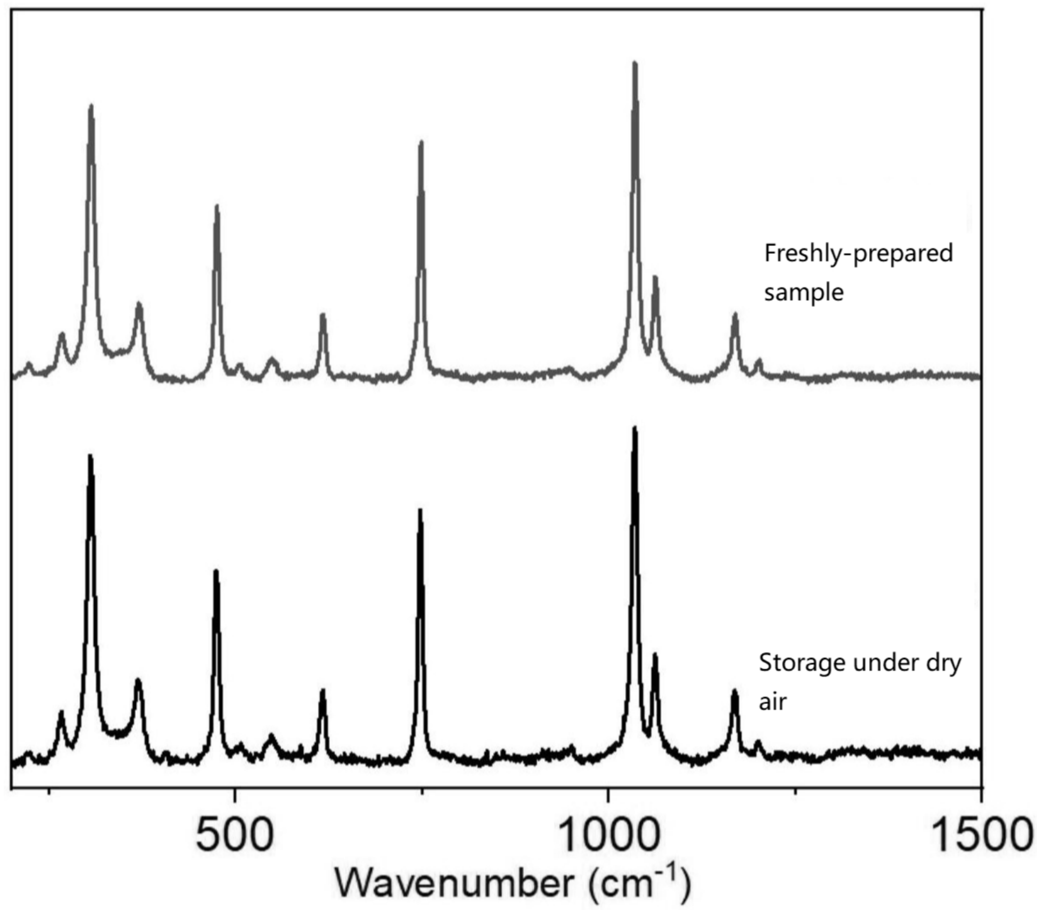
Freshly-prepared sample
Storage under dry air
500
1000
1500
Wavenumber (cm-1)

FIG.4

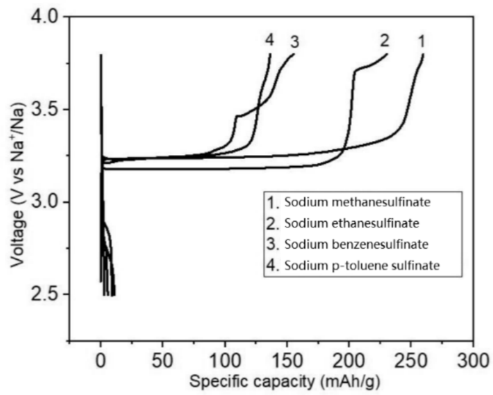
Voltage (V vs Na+/Na)
Specific capacity (mAh/g)
1. Sodium methanesulfinate
2. Sodium ethanesulfinate
3. Sodium benzenesulfinate
4. Sodium p-toluene sulfinate

a

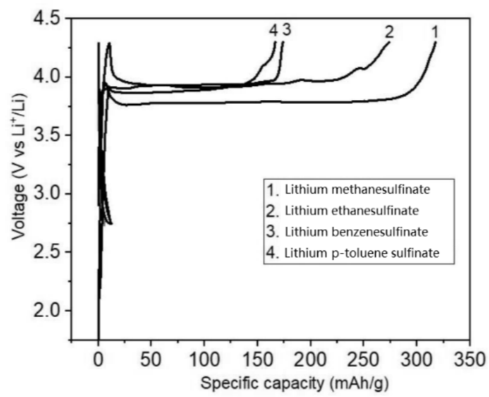
Voltage (V vs Li+/Li)
Specific capacity (mAh/g)
1. Lithium methanesulfinate
2. Lithium ethanesulfinate
3. Lithium benzenesulfinate
4. Lithium p-toluene sulfinate

b

FIG.5

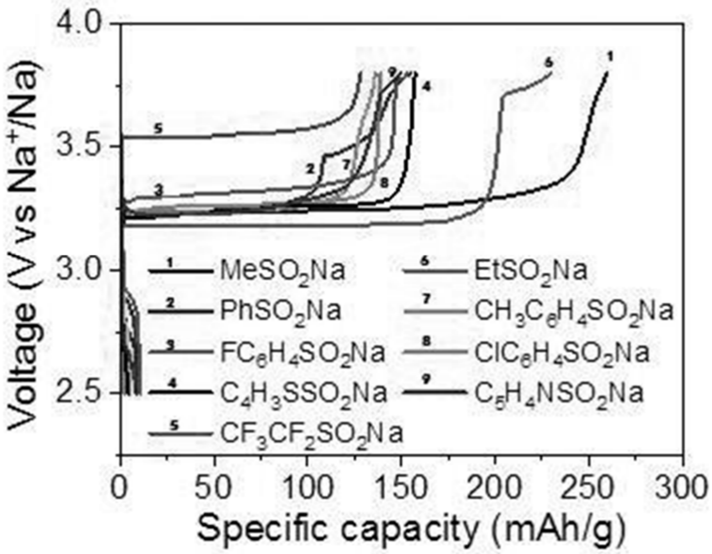


FIG.6

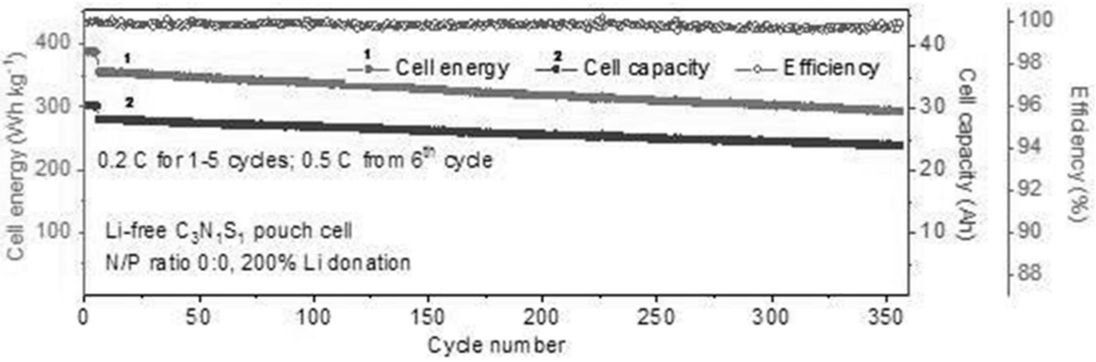


FIG.7

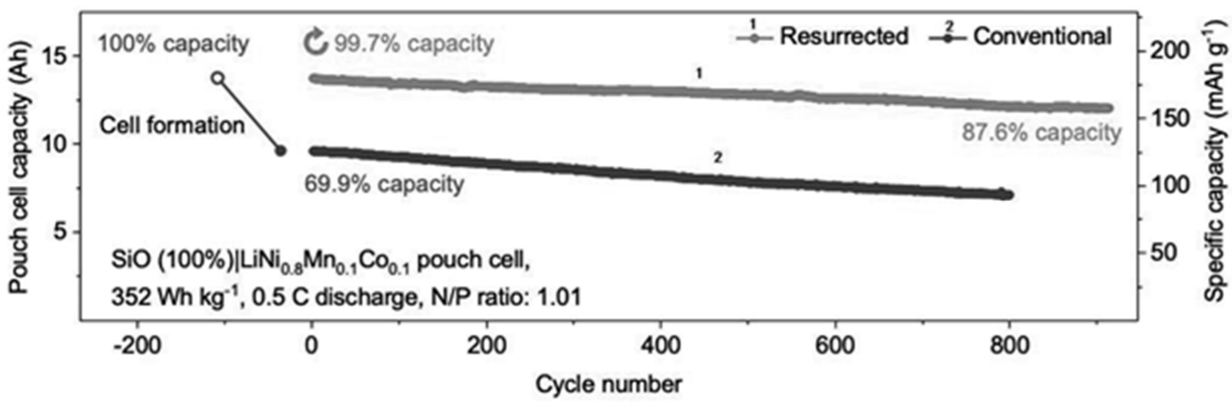


FIG.8

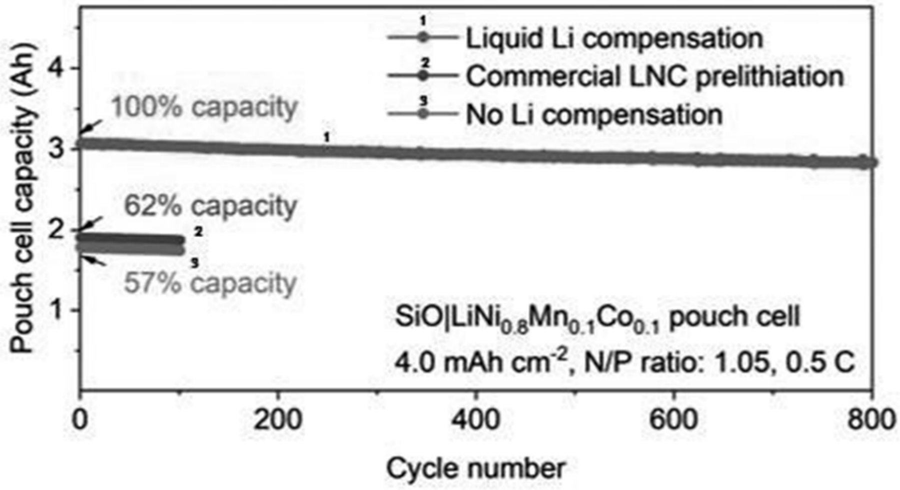
Pouch cell capacity (Ah)
Liquid Li compensation
Commercial LNC prelithiation
No Li compensation
100% capacity
62% capacity
57% capacity
SiO|LiNi0.8Mn0.1Co0.1 pouch cell
4.0 mAh cm-2, N/P ratio: 1.05, 0.5 C
Cycle number

FIG.9

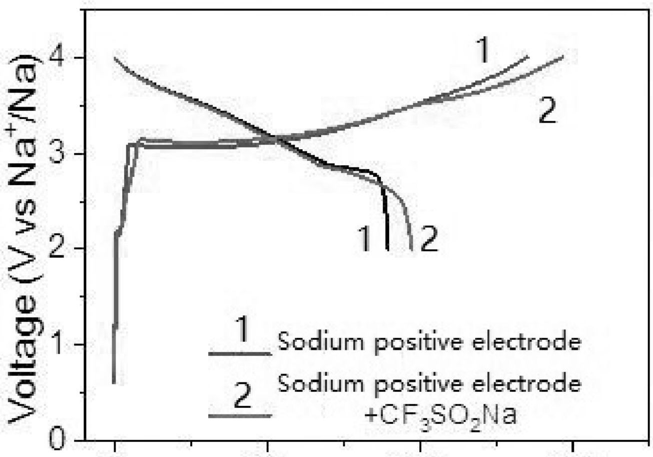
Voltage (V vs Na+/Na)
Sodium positive electrode
Sodium positive electrode
+CF3SO2Na
Specific capacity (mAh/g)

FIG.10

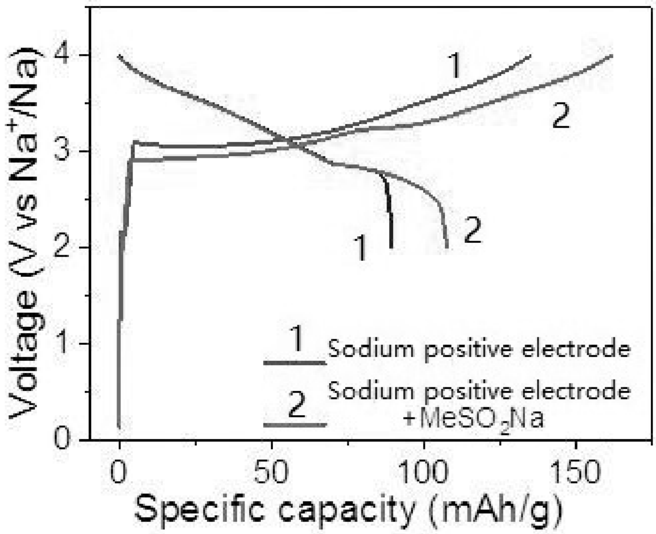


FIG.11

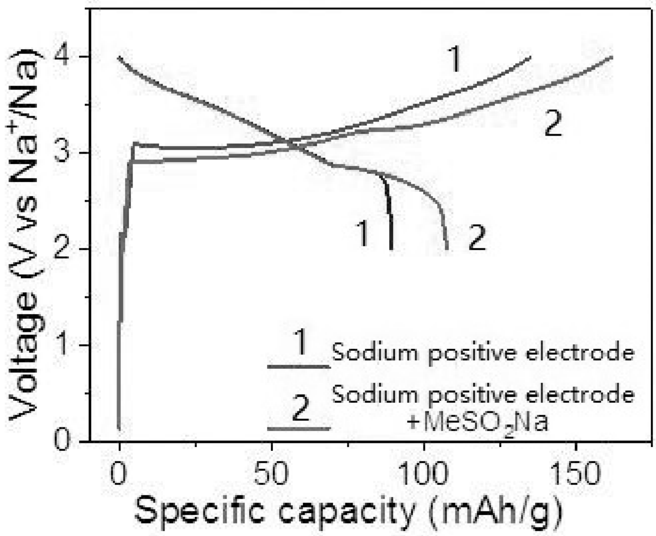


FIG.12

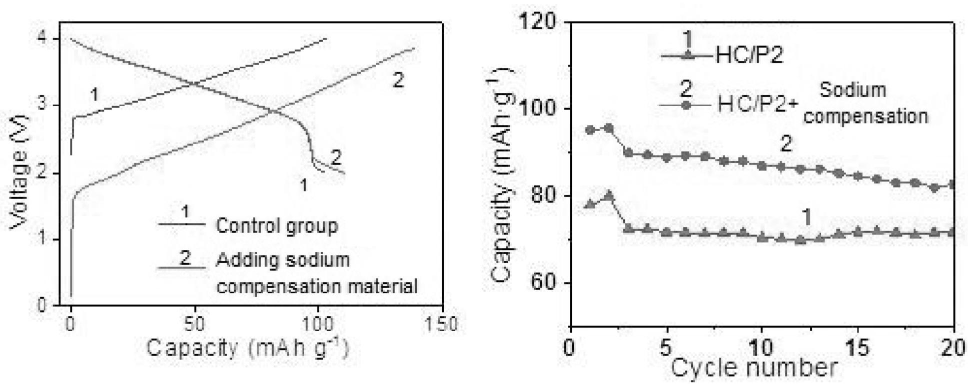


FIG.13

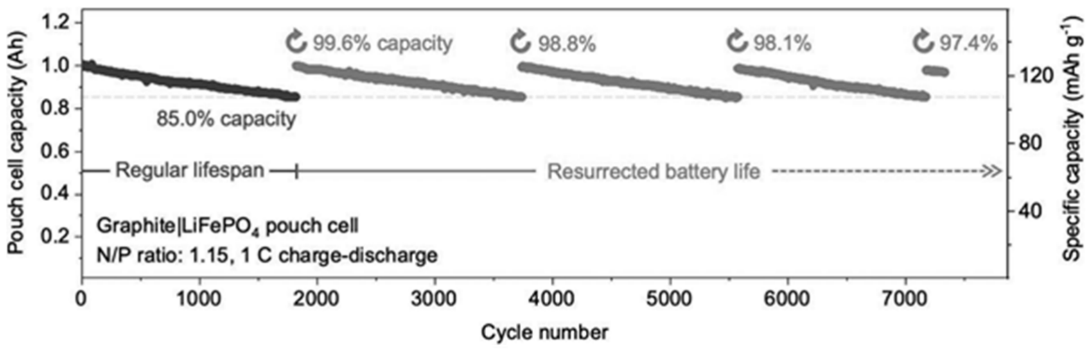
Pouch cell capacity (Ah)
1.2
1.0
0.8
0.6
0.4
0.2
99.6% capacity
98.8%
98.1%
97.4%
85.0% capacity
Regular lifespan
Resurrected battery life
Graphite|LiFePO4 pouch cell
N/P ratio: 1.15, 1 C charge-discharge
Specific capacity (mAh g-1)
120
80
40
0
1000
2000
3000
4000
5000
6000
7000
Cycle number

FIG.14

## INTERNATIONAL SEARCH REPORT

International application No.
**PCT/CN2024/084855**

**A. CLASSIFICATION OF SUBJECT MATTER**

H01M10/058(2010.01)i

According to International Patent Classification (IPC) or to both national classification and IPC

**B. FIELDS SEARCHED**

Minimum documentation searched (classification system followed by classification symbols)

IPC:H01M

Documentation searched other than minimum documentation to the extent that such documents are included in the fields searched

Electronic data base consulted during the international search (name of data base and, where practicable, search terms used)

CNTXT, ENTXT, ENTXTC, DWPI, CNKI: 预嵌锂, 预锂, 补锂, 预嵌钠, 预钠, 补钠, 亚磺酸, pre, lithium, supplement+, sodium, sulfinate

**C. DOCUMENTS CONSIDERED TO BE RELEVANT**

| Category* | Citation of document, with indication, where appropriate, of the relevant passages | Relevant to claim No. |
|---|---|---|
| PX | CN 116487713 A (FUDAN UNIVERSITY) 25 July 2023 (2023-07-25) description, paragraphs 4-80 | 1-21 |
| X | CN 114447333 A (TIANJIN ZHONGJIN NEW ENERGY RESEARCH INSTITUTE CO., LTD.) 06 May 2022 (2022-05-06) description, paragraphs 7-78 | 1-21 |
| A | CN 113097453 A (RISESUN MGL NEW ENERGY TECHNOLOGY CO., LTD.) 09 July 2021 (2021-07-09) entire document | 1-21 |
| A | US 2021384558 A1 (NIPPON TELEGRAPH AND TELEPHONE CORP.) 09 December 2021 (2021-12-09) entire document | 1-21 |

☐ Further documents are listed in the continuation of Box C.　☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **30 May 2024** | **12 June 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**
**Information on patent family members**

International application No.
**PCT/CN2024/084855**

| Patent document cited in search report | | | Publication date (day/month/year) | Patent family member(s) | | | Publication date (day/month/year) |
|---|---|---|---|---|---|---|---|
| CN | 116487713 | A | 25 July 2023 | None | | | |
| CN | 114447333 | A | 06 May 2022 | CN | 114447333 | B | 26 March 2024 |
| CN | 113097453 | A | 09 July 2021 | None | | | |
| US | 2021384558 | A1 | 09 December 2021 | WO | 2020105431 | A1 | 28 May 2020 |
| | | | | JP | 2020087585 | A | 04 June 2020 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 116470165 A
- CN 107845829 A
- CN 110165218 A